# EUROPÄISCHE PATENTANMELDUNG

(11) **EP 0 778 274 A1**
(43) Veröffentlichungstag der Anmeldung: **11.06.1997**
(21) Anmeldenummer: 96250208.4
(22) Anmeldetag: 24.09.1996
(51) Int. Cl.: C07D 307/81, A61K 31/34

(54) **Amidinohydrazone vom Benzo(b)furan abgeleiteter Ketone, Verfahren zu deren Herstellung und diese Verbindungen enthaltende Arzneimittel**

(30) Priorität: 07.12.1995 DE 19547263
(71) Anmelder: Helopharm G. Petrik GmbH, 13403 Berlin (DE)
(72) Erfinder: Richter, Peter H., Prof. Dr., 17489 Greifswald (DE); Elsner, Martin, Dr., 33607 Bielefeld (DE); Vogt, Barbara, Dipl.-Biol., 13465 Berlin (DE)
(74) Vertreter: Wablat, Wolfgang, Dr.Dr.

(57) **Zusammenfassung**

Beschrieben sind neue Amidinohydrazone vom Benzo[*b*]furan abgeleiteter Ketone der allgemeinen Formel I 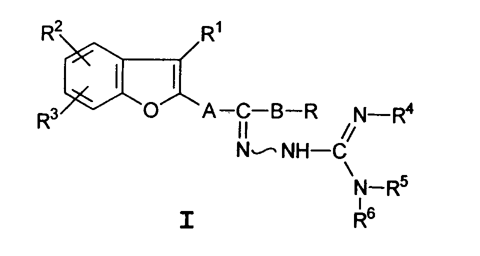 und deren pharmazeutisch annehmbare Salze, ein Verfahren zu ihrer Herstellung und diese Verbindungen enthaltende Arzneimittel.
Die beschriebenen Verbindungen zeigen verbesserte Klasse III-antiarrhythmische Wirkungen.

## Beschreibung

Die Erfindung betrifft neue Amidinohydrazone, Verfahren zu ihrer Herstellung und diese Verbindungen enthaltende Arzneimittel.

In der Therapie von Herzrhythmusstörungen werden eine Reihe von Verbindungen angewendet, die in ihrer chemischen Struktur und pharmakologischen Wirkungsweise sehr unterschiedlich sind. Zur Unterscheidung der verschiedenen Antiarrhythmika wurde 1970 von Vaughan Williams ein Klassifizierungssystem vorgeschlagen, das im wesentlichen auf der unterschiedlichen Beeinflussung der transmembranären Ionenströme durch die Substanzen der verschiedenen Stoffklassen basiert.

Verbindungen der Klasse I, wie beispielsweise Flecainid (INN), Lidocain (INN) und Propafenon (INN) wirken lokalanästhetisch auf Nerven- und Myokardmembranen durch Hemmung des transmembranären Natriumeinstromes. Sie verlangsamen dadurch die Reizleitung, wodurch wiederum die Weiterleitung von vorzeitigen Herzschlägen verringert wird. Außerdem wird die Tendenz geschädigter Zellen zur Aussendung vorzeitiger Herzschläge unterdrückt.

Die Verbindungen der Klasse II sind sogenannte β-Blocker. Als Beispiel sei hier Propranolol (INN) genannt.

Die Wirkung der β-Blocker als Antiarrhythmika besteht in einer Abschwächung bzw. Blockade adrenerger Effekte, die ihrerseits mit den transmembranären Ionenströmen interferieren.

Klasse III-Antiarrhythmika sind in ihrer Wirkung weitestgehend unabhängig von einer Hemmung des transmembranären Natriumstromes. Sie haben keinen oder nur geringen Einfluß auf das Ruhepotential der Herzzellen und die Reizleitung. Diese Verbindungen verlängern die Dauer des kardialen Aktionspotentials und verlängern so die Refraktärzeit, d. h. das Zeitintervall, in dem die Herzzellen nicht erregbar sind. Der zugrundeliegende Mechanismus besteht in einer Beeinflussung des transmembranären repolarisierenden Kaliumausstromes.

Als Klasse IV-Antiarrhythmika werden herzwirksame Calciumantagonisten wie Verapamil (INN) oder Diltiazem (INN) bezeichnet. Diese Verbindungen hemmen den langsamen Calciumeinstrom im Beginn der Erregung und unterdrücken so die Erregungsbildung und -ausbreitung sogenannter langsamer Aktionspotentiale, die unter bestimmten pathologischen Bedingungen im Herzmuskel entstehen.

Im Zusammenhang mit den erfindungsgemäßen Verbindungen ist auf die Klasse III-Antiarrhythmika Bezug zu nehmen.

Als Prototypen der Klasse III-Antiarrhythmika zählen Amiodaron (INN) und Sotalol (INN), die aber beide keine "reinen" Klasse III-Antiarrhythmika sind. Sotalol hat außerdem β-blockierende Eigenschaften und somit auch eine Klasse II-Wirkung, Amiodaron zeigt zusätzliche Wirkungen auf Natrium- und Calciumkanäle und hat auch α- und β-adrenolytische Wirkungen. Den wertvollen Eigenschaften dieser beiden Antiarrhythmika stehen gravierende Nebenwirkungen gegenüber, die ihren Einsatz einschränken oder sogar verbieten. Dazu gehören gastrointestinale Beschwerden wie Übelkeit, Erbrechen, Völlegefühl, Verstopfung aber auch zerebrale Störungen wie Kopfschmerzen, Schwindel, Sehstörungen, Schlafstörungen und dermatologische Probleme wie Hyperpigmentierung der Haut und Sonnenlichtempfindlichkeit. Als gravierendste und potentiell letale Nebenwirkungen können Lungenfibrosen und Leberschädigungen sowie *pro*arrhythmische Effekte in Form von Blockierungen der AV-Überleitung und *Torsade de pointes* auftreten, die einen Abbruch der Therapie erforderlich machen.

Während dem Amiodaron verwandte Benzo[*b*]furanyl-Ketone bereits auf ihre antiarrhythmischen Eigenschaften hin geprüft wurden, sind antiarrhythmisch wirksame Amidinohydrazone vom Benzo[*b*]furan abgeleiteter Ketone bisher nicht bekannt. In der Literatur sind lediglich das 5-Amidinobenzo[*b*]furan-2-carboxaldehydamidinohydrazondihydrochlorid als trypanozid (Dann O., Char H., Greissmeier H.: Liebigs Ann. Chem *1982*, 1836) sowie das Bis-(7-Hydroxybenzo[*b*]furan-2-yl)ketonamidinohydrazon-hydrochlorid (Kaken Pharm Co Ltd: JP 59 206 378) als antiviral beschrieben.

Es besteht daher ein dringender Bedarf an neuartigen antiarrhythmisch wirksamen Substanzen.

Aufgabe der vorliegenden Erfindung ist es nun, Verbindungen zur Verfügung zu stellen, welche ein besseres Wirksamkeits-/Nebenwirkungsverhältnis als die bisher verfügbaren Klasse III-Antiarrhythmika aufweisen.

Erfindungsgemäß wird diese Aufgabe durch die Schaffung neuer Amidinohydrazone der allgemeinen Formel I 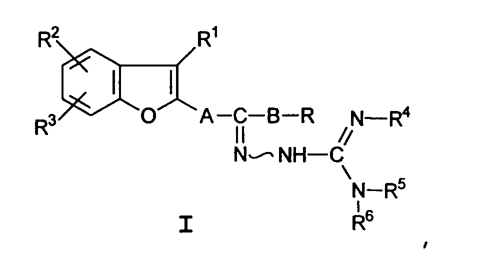 worin
R eine lineare oder verzweigte Alkyl- oder Dialkylaminoethylgruppe mit jeweils bis zu 6 C-Atomen oder einen der Reste 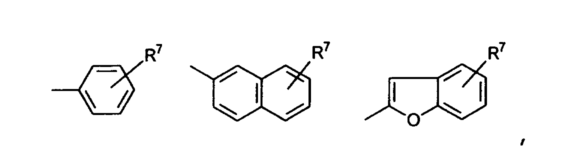
worin
R⁷ ein Wasserstoffatom, ein Halogenatom, eine lineare oder verzweigte Alkyl- oder Alkoxygruppe mit bis zu 6 C-Atomen, eine Aralkyl- oder Aralkoxygruppe mit bis zu 9 C-Atomen, eine Cyano-, eine Nitro-, eine Methansulfonamido-, eine Acetylamino-, eine Trifluormethyl-, eine Trifluormethoxy-, eine Amino- oder eine (1*H*-Imidazol-1-yl)-Gruppe ist,
   darstellt,
A und B unabhängig voneinander die Bedeutung (CH₂)ₙ oder (CH=CH)ₘ haben, wobei n = 0, 1 oder 2 und m = 0 oder 1 ist,
R¹ ein Wasserstoffatom, ein Amino-, ein linearer oder verzweigter Alkylrest mit bis zu 6 C-Atomen, ein Aralkylrest mit bis zu 9 C-Atomen, ein Methansulfonamido-, ein Acetylamino-, ein Cyano-, ein (1*H*-Imidazol-1-yl)-Rest oder einer der Reste 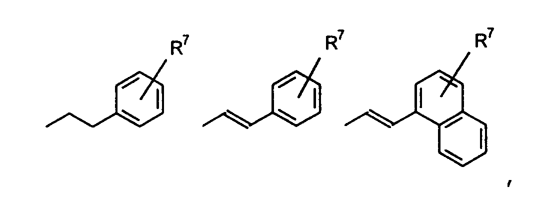
worin
R⁷ die oben angegebene Bedeutung hat, ist,
R² und R³ unabhängig voneinander ein Wasserstoffatom, eine lineare oder verzweigte Alkyl- oder Alkoxygruppe mit bis zu 6 C-Atomen, eine Aralkyl- oder Aralkoxygruppe mit bis zu 9 C-Atomen, ein Halogenatom, eine Cyano-, eine Nitro-, eine Methansulfonamido-, eine Acetylamino-, eine Trifluormethyl-, eine Trifluormethoxy-, eine Amino- oder eine (1*H*-Imidazol-1-yl)-Gruppe sind,
R⁴ ein Wasserstoffatom, eine lineare oder verzweigte Alkylgruppe mit bis zu 6 C-Atomen, eine Aralkylgruppe mit bis zu 9 C-Atomen oder einen der Reste 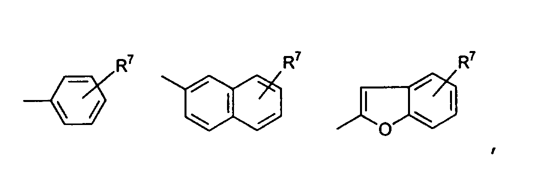
worin
R⁷ die oben angegebene Bedeutung hat, darstellt,
R⁵ und R⁶ unabhängig voneinander ein Wasserstoffatom, einen linearen oder verzweigten Alkyl-, Alkanoyl- oder Alkansulfonylrest mit jeweils bis zu 6 C-Atomen, einen Aralkylrest mit bis zu 9 C-Atomen, einen (4-Methylphenyl)sulfonyl-, einen Trifluoracetyl- oder einen der Reste 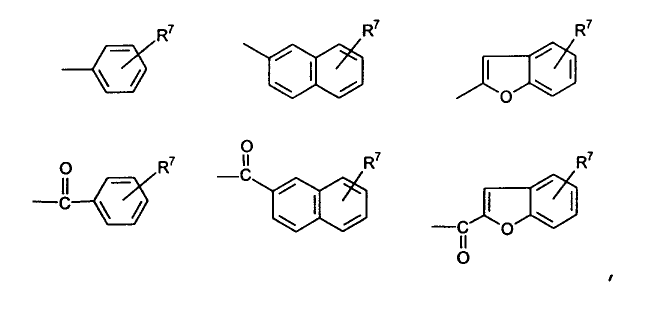
worin
R⁷ die oben angegebene Bedeutung hat, bedeuten,
oder R⁴ und R⁵ zusammen ein Ethylen- oder Propylenfragment darstellen,
oder R⁵ und R⁶ zusammen mit dem N-Atom einen Piperidino-, Morpholino- oder Piperazinorest bedeuten,
und die gewellte Bindung in der Amidinohydrazonstruktur bedeutet, daß die Verbindungen als (Z)- oder (E)-Isomere oder als Isomerengemische vorliegen,
sowie deren Salze mit einer oder mehreren physiologisch verträglichen Säuren wie Mineralsäuren, linearen oder verzweigten Alkan- oder Alkensäuren oder Alkansulfonsäuren mit bis zu 6 C-Atomen oder Arencarbonsäuren, wobei die organischen Säuren gegebenenfalls mit Halogen-, Amino-, Dialkylamino- (mit bis zu 6-C-Atomen), Hydroxy- und Carboxy-Resten substituiert sind,
gelöst.

Bevorzugt sind Verbindungen, bei denen A und B entfallen und R Phenyl, 2-Benzo[*b*]furanyl oder 2-Naphthyl, substituiert durch R⁷, welches Wasserstoff, *n*-Alkyl, *verzweigt*-Alkyl, Aralkyl, Alkoxy, Aralkoxy, Halogen, Cyan, Nitro, Methansulfonamido, Acetylamino, Trifluormethyl, Trifluormethoxy, Amin · HX oder (1*H*-Imidazol-1-yl) darstellt, ist.
Bevorzugt sind hierbei Verbindungen, worin R⁴, R⁵ und R⁶ gleichzeitig Wasserstoff darstellen.
Weiterhin bevorzugt sind hierbei Verbindungen, worin R⁴ und R⁵ gleichzeitig Wasserstoff und R⁶ *n*-Alkyl, *verzweigt*-Alkyl, Aralkyl, *n*-Alkansulfonyl, *verzweigt-*Alkansulfonyl, *n*-Alkanoyl, *verzweigt*-Alkanoyl, (4-Methylphenyl)sulfonyl, Trifluoracetyl, Phenyl, 2-Benzo[*b*]furanyl oder 2-Naphthyl, gegebenenfalls substituiert durch R⁷, das Wasserstoff, *n*-Alkyl, *verzweigt*-Alkyl, Aralkyl, Alkoxy, Aralkoxy, Halogen, Cyan, Nitro, Methansulfonamido, Acetylamino, Trifluormethyl, Trifluormethoxy, Amin · HX oder (1*H*-Imidazol-1-yl) darstellt, sind.
Bevorzugt sind hierbei weiterhin Verbindungen, worin R⁴ Wasserstoff und R⁵ und R⁶ gleichzeitig *n*-Alkyl, *verzweigt*-Alkyl, Aralkyl oder zusammen mit dem sie tragenden Stickstoffatom Piperidino, Morpholino oder Piperazino · HX darstellen. Bevorzugt sind hierbei ferner noch Verbindungen, worin R⁴ und R⁵ zusammen eine Ethylen- oder Propylengruppe und R⁶ Wasserstoff darstellen.
Bevorzugt sind hierbei weiterhin noch Verbindungen, worin R⁴ und R⁵ Wasserstoff und R⁶ Benzoyl, 2-Benzo[*b*]furanoyl oder 2-Naphthoyl, in dem R⁷ Wasserstoff, *n*-Alkyl, *verzweigt*-Alkyl, Aralkyl, Alkoxy, Aralkoxy, Halogen, Cyan, Nitro, Methansulfonamido, Acetylamino, Trifluormethyl, Trifluormethoxy, Amin · HX oder (1*H*-Imidazol-1-yl) darstellen, sind.

Bevorzugt sind Verbindungen, bei denen A entfällt, B eine Ethenylgruppe (m = 1) und R Phenyl, 2-Benzo[*b*]furanyl oder 2-Naphthyl, substituiert durch R⁷, das Wasserstoff, *n*-Alkyl, *verzweigt*-Alkyl, Aralkyl, Alkoxy, Aralkoxy, Halogen, Cyan, Nitro, Methansulfonamido, Acetylamino, Trifluormethyl, Trifluormethoxy, Amin · HX oder (1*H*-Imidazol-1-yl) darstellt, sind. Besonders bevorzugt sind hierbei Verbindungen, worin R⁴, R⁵ und R⁶ gleichzeitig Wasserstoff darstellen.

Bevorzugt sind ferner Verbindungen, bei denen B entfällt, A eine Ethenylgruppe (m = 1) und R Phenyl, 2-Benzo[*b*]furanyl oder 2-Naphthyl, substituiert durch R⁷, das Wasserstoff, *n*-Alkyl, *verzweigt*-Alkyl, Aralkyl, Alkoxy, Aralkoxy, Halogen, Cyan, Nitro, Methansulfonamido, Acetylamino, Trifluormethyl, Trifluormethoxy, Amin · HX oder (1*H*-Imidazol-1-yl) darstellt, sind. Besonders bevorzugt sind hierbei Verbindungen, worin R⁴, R⁵ und R⁶ gleichzeitig Wasserstoff darstellen.

Bevorzugt sind auch Verbindungen, bei denen A entfällt, B eine oder zwei Methylengruppen (n = 1, 2) und R Phenyl, 2-Benzo[*b*]furanyl oder 2-Naphthyl, substituiert durch R⁷, das Wasserstoff, *n*-Alkyl, *verzweigt*-Alkyl, Aralkyl, Alkoxy, Aralkoxy, Halogen, Cyan, Nitro, Methansulfonamido, Acetylamino, Trifluormethyl, Trifluormethoxy, Amin · HX oder (1*H*-Imidazol-1-yl) darstellt, sind. Besonders bevorzugt sind hierbei Verbindungen, worin R⁴, R⁵ und R⁶ gleichzeitig Wasserstoff darstellen.

Bevorzugt sind außerdem Verbindungen, bei denen B entfällt, A eine oder zwei Methylengruppen (n = 1, 2) und R Phenyl, 2-Benzo[*b*]furanyl oder 2-Naphthyl, substituiert durch R⁷, das Wasserstoff, *n*-Alkyl, *verzweigt*-Alkyl, Aralkyl, Alkoxy, Aralkoxy, Halogen, Cyan, Nitro, Methansulfonamido, Acetylamino, Trifluormethyl, Trifluormethoxy, Amin · HX oder (1*H*-Imidazol-1-yl) darstellt, sind. Besonders bevorzugt sind hierbei Verbindungen, worin R⁴, R⁵ und R⁶ gleichzeitig Wasserstoff darstellen.

Besonders bevorzugt sind Verbindungen, welche als reines (Z)-Isomer vorliegen.

Besonders bevorzugt sind Verbindungen, welche als reines (E)-Isomer vorliegen.

Insbesondere sind bevorzugt:
(E)-2-Benzoylbenzo[*b*]furanamidinohydrazon-hydrochlorid **39**
(Z)-2-Benzoylbenzo[*b*]furanamidinohydrazon-hydrochlorid **40**
(Z/E)-2-Benzoylbenzo[*b*]furanamidinohydrazon-hydrochlorid **39/40**
(Z/E)-2-(4-Methylbenzoyl)benzo[*b*]furanamidinohydrazon-hydrochlorid **41**
(Z/E)-2-(4-Methoxybenzoyl)benzo[*b*]furanamidinohydrazon-hydrochlorid **42**
(Z/E)-2-(4-Methansulfonamidobenzoyl)benzo[*b*]furanamidinohydrazon-hydrathydrochlorid **43**
(Z/E)-2-(4-Aminobenzoyl)benzo[*b*]furanamidinohydrazondihydrochlorid **44**
(Z/E)-2-(4-Brombenzoyl)benzo[*b*]furanamidinohydrazon-hydrochlorid **45**
(Z/E)-2-(4-Chlorbenzoyl)benzo[*b*]furanamidinohydrazon-hydrochlorid **46**
(Z/E)-2-(4-Nitrobenzoyl)benzo[*b*]furanamidinohydrazon-hydrochlorid **47**
(Z/E)-2-(4-Cyanbenzoyl)benzo[*b*]furanamidinohydrazon-hydrochlorid **48**
(Z/E)-5-Brom-2-(4-cyclohexylbenzoyl)benzo[*b*]furanamidinohydrazon-hydrochlorid **49**
(Z/E)-2-(3-Nitrobenzoyl)benzo[*b*]furanamidinohydrazon-hydrochlorid **50**
(Z/E)-2-Benzoyl-5-brombenzo[*b*]furanamidinohydrazon-hydrochlorid **51**
(Z/E)-5-Brom-2-(4-chlorbenzoyl)benzo[*b*]furanamidinohydrazon-hydrochlorid **52**
(Z/E)-5-Brom-2-(4-brombenzoyl)benzo[*b*]furanamidinohydrazon-hydrochlorid **53**
(Z/E)-5-Brom-2-(4-cyanbenzoyl)benzo[*b*]furanamidinohydrazon-hydrochlorid **54**
(Z/E)-2-(4-Acetylaminobenzoyl)-5-brombenzo[*b*]furanamidinohydrazon-hydrochlorid **55**
(Z/E)-2-Benzoyl-5-nitrobenzo[*b*]furanamidinohydrazon-hydrochlorid **56**
(Z/E)-2-Benzoyl-5-methansulfonamidobenzo[*b*]furanamidinohydrazon-hydrochlorid **57**
(Z/E)-5-Brom-2-(4-nitrobenzoyl)benzo[*b*]furanamidinohydrazon-hydrochlorid **58**
(Z/E)-2-(4-Cyanbenzoyl)-5-nitrobenzo[*b*]furanamidinohydrazon-hydrochlorid **59**
(Z/E)-2-Benzoyl-5-cyanbenzo[*b*]furanamidinohydrazon-hydrochlorid **60**
(Z/E)-5-Brom-2-(3-nitrobenzoyl)benzo[*b*]furanamidinohydrazon-hydrochlorid **61**
(Z/E)-2-Benzoyl-5,7-diiodbenzo[*b*]furanamidinohydrazon-hydrochlorid **62**
(Z/E)-2-Benzoyl-5,7-dibrombenzo[*b*]furanamidinohydrazon-hydrochlorid **63**
(Z/E)-5-Brom-2-(4-methylbenzoyl)benzo[*b*]furanamidinohydrazon-hydrochlorid **64**
(Z/E)-2-(4-Fluorbenzoyl)benzo[*b*]furanamidinohydrazon-hydrochlorid **65**
(Z/E)-2-(3,4-Dimethoxybenzoyl)benzo[*b*]furanamidinohydrazon-hydrochlorid **66**
(Z/E)-2-(2-Fluorbenzoyl)benzo[*b*]furanamidinohydrazon-hydrochlorid **67**
(Z/E)-2-(4-Trifluormethoxybenzoyl)benzo[*b*]furanamidinohydrazon-hydrochlorid **68**
(Z/E)-2-(4-Trifluormethylbenzoyl)benzo[*b*]furanamidinohydrazon-hydrochlorid **69**
(Z/E)-2-(3-Trifluormethoxybenzoyl)benzo[*b*]furanamidinohydrazon-hydrochlorid **70**
(Z/E)-2-(3-Trifluormethylbenzoyl)benzo[*b*]furanamidinohydrazon-hydrochlorid **71**
(Z/E)-5,7-Diiod-2-(4-trifluormethylbenzoyl)benzo[*b*]furanamidinohydrazon-hydrochlorid **72**
(Z/E)-5-Nitro-2-(3-trifluormethoxybenzoyl)benzo[*b*]furanamidinohydrazon-hydrochlorid **73**
(Z/E)-5-Chlor-2-(4-trifluormethylbenzoyl)benzo[*b*]furanamidinohydrazon-hydrochlorid **74**
(Z/E)-5-Nitro-2-(4-trifluormethylbenzoyl)benzo[*b*]furanamidinohydrazon-hydrochlorid **75**
(Z/E)-5-Nitro-2-(4-trifluormethoxybenzoyl)benzo[*b*]furanamidinohydrazon-hydrochlorid **76**
(Z/E)-5-Nitro-2-(3-trifluormethylbenzoyl)benzo[*b*]furanamidinohydrazon-hydrochlorid **77**
(Z/E)-2-Benzoyl-5-chlorbenzo[*b*]furanamidinohydrazon-hydrochlorid **78**
(Z/E)-2-Benzoyl-7-methoxybenzo[*b*]furanamidinohydrazon-hydrochlorid **79**
(Z/E)-5-Methansulfonamido-2-(3-trifluormethylbenzoyl)benzo[*b*]furanamidinohydrazon-hydrochlorid **80**
(Z/E)-1-(Benzo[*b*]furan-2-yl)-3-(4-methansulfonamidophenyl)prop-2-en-1-onamidinohydrazon-hydrochlorid **81**
(Z/E)-1-(Benzo[*b*]furan-2-yl)-3-phenylprop-2-en-1-onamidinohydrazon-hydrochlorid **82**
(Z/E)-1-(Benzo[*b*]furan-2-yl)-3-(4-fluorphenyl)prop-2-en-1-onamidinohydrazon-hydrochlorid **83**
(Z/E)-1-(Benzo[*b*]furan-2-yl)-3-(4-chlorphenyl)prop-2-en-1-onamidinohydrazon-hydrochlorid **84**
(Z/E)-1-(Benzo[*b*]furan-2-yl)-3-(4-nitrophenyl)prop-2-en-1-onamidinohydrazon-hydrochlorid **85**
(Z/E)-1-(Benzo[*b*]furan-2-yl)-3-(4-methoxyphenyl)prop-2-en-1-onamidinohydrazon-hydrochlorid **86**
(Z/E)-1-(Benzo[*b*]furan-2-yl)-3-(3-bromphenyl)prop-2-en-1-onamidinohydrazon-hydrochlorid **87**
(Z/E)-1-(Benzo[*b*]furan-2-yl)-3-(3-nitrophenyl)prop-2-en-1-onamidinohydrazon-hydrochlorid **88**
(Z/E)-1-(Benzo[*b*]furan-2-yl)-3-(4-trifluormethylphenyl)prop-2-en-1-onamidinohydrazon-hydrochlorid **89**
(Z/E)-1-(Benzo[*b*]furan-2-yl)-3-phenylpropan-1-onamidinohydrazon-hydrochlorid **90**
(Z/E)-1-(Benzo[*b*]furan-2-yl)-3-(4-chlorphenyl)propan-1-onamidinohydrazon-hydrochlorid **91**
(Z/E)-1-(Benzo[*b*]furan-2-yl)-3-(4-methoxyphenyl)propan-1-onamidinohydrazon-hydrochlorid **92**
(Z/E)-1-(Benzo[*b*]furan-2-yl)-3-(3-methansulfonamidophenyl)propan-1-onamidinohydrazon-hydrochlorid **93**
(Z/E)-3-(Benzo[*b*]furan-2-yl)-1-phenylprop-2-en-1-onamidinohydrazon-hydrochlorid **94**
(Z/E)-3-(Benzo[*b*]furan-2-yl)-1-(3-methansulfonamidophenyl)prop-2-en-1-onamidinohydrazon-hydrochlorid **95**
(Z/E)-3-(Benzo[*b*]furan-2-yl)-1-(4-methansulfonamidophenyl)prop-2-en-1-onamidinohydrazon-hydrochlorid **96**
(Z/E)-3-(Benzo[*b*]furan-2-yl)-1-(3-methansulfonamidophenyl)propan-1-onamidinohydrazon-hydrochlorid **97**
(Z/E)-3-(Benzo[*b*]furan-2-yl)-1-(4-methansulfonamidophenyl)propan-1-onamidinohydrazon-hydrochlorid **98**
(Z/E)-2-Benzoylbenzo[*b*]furan-*N*^{*3*},*N*^{*3*}-dimethylamidinohydrazon-hydrochlorid **99**
(Z/E)-2-Benzoylbenzo[*b*]furan-*N*^{*3*}-phenyl-amidinohydrazonium-nitrat **100**
(Z/E)-2-Benzo[*b*]furan-(4-methansulfonamidobenzoyl)-*N*^{*3*}-phenylamidinohydrazonium-nitrat **101**
(Z/E)-2-Benzoylbenzo[*b*]furan-*N*^{*3*}-(pentamethylen)hydrazon-hydrochlorid **102**
(Z/E)-2-Benzoylbenzo[*b*]furan-*N*^{*3*}-prop-2-yl-amidinohydrazon-hydroiodid **103**
Bis-(Benzo[*b*]furan-2-yl)-ketonamidinohydrazon-hydrochlorid **104**
(Z/E)-(5-Brombenzo[*b*]furan-2-yl)-(naphth-2-yl)ketonamidinohydrazon-hydrochlorid **105**
(Z/E)-(Benzo[*b*]furan-2-yl)-(5-brombenzo[b]furan-2-yl)ketonamidinohydrazon-hydrochlorid **106**
(Z/E)-1-(Benzo[*b*]furan-2-yl)-3-dimethylaminopropan-1-onamidinohydrazon-hydrochlorid **107**
(Z/E)-2-Acetylbenzo[*b*]furanamidinohydrazon-hydrochlorid **108**
(Z/E)-Benzo[*b*]furan-2-yl)-(5,7-dibrombenzo[*b*]furan-2-yl)ketonamidinohydrazon-hydrochlorid **109**
(Z/E)-1-(5-Brombenzo[*b*]furan-2-yl)propan-1-onamidinohydrazon-hydrochlorid **110**
(Z/E)-2-Phenylacetylbenzo[*b*]furanamidinohydrazon-hydrochlorid **111**
(Z/E)-5-Chlor-2-phenylacetylbenzo[*b*]furanamidinohydrazon-hydrochlorid **112**
(Z/E)-5-Brom-2-phenylacetylbenzo[*b*]furanamidinohydrazon-hydrochlorid **113**
(Z/E)-1-(Benzo[*b*]furan-2-yl)propan-1-onamidinohydrazon-hydrochlorid **114**
(Z/E)-2-Benzoylbenzo[*b*]furan-*N*^{*2*}-(imidazolin-2-yl)hydrazon-hydrochlorid **115**
(Z/E)-2-Benzoyl-5,7-dibrombenzo[*b*]furan-*N*^{*2*}-(imidazolin-2-yl)hydrazon-hydrochlorid **116**
(Z/E)-3-Amino-2-benzoyl-benzo[*b*]furanamidinohydrazon-hydrochloridhydrat **117**
(Z/E)-2-Benzoyl-3-methylbenzo[*b*]furanamidinohydrazon-hydrochlorid **118**
(Z/E)-2-Benzoyl-5-benzyloxy-3-phenethylbenzo[*b*]furanamidinohydrazon-hydrochlorid **119**
(Z/E)-2-Benzoyl-7-benzyloxy-3-methylbenzo[*b*]furanamidinohydrazon-hydrochlorid **120**
(Z/E)-2-Benzoyl-3-(2-(naphth-1-yl)ethen-1-yl)benzo[*b*]furanamidinohydrazon-hydrochlorid **121**
(Z/E)-2-Benzoyl-7-benzyloxy-3-phenethylbenzo[*b*]furanamidinohydrazon-hydrochlorid **122**
(Z/E)-2-Benzoyl-3-methyl-5-nitrobenzo[*b*]furanamidinohydrazon-hydrochlorid **123**
(Z/E)-2-Benzoylbenzo[*b*]furan-*N*^{*3*}-benzoylamidinohydrazon-hydrochlorid **124**
(Z/E)-2-Benzoylbenzo[*b*]furan-*N*^{*3*}-(4-methansulfonamidobenzoyl)amidinohydrazon-hydrochlorid **125**
(Z/E)-2-Benzoylbenzo[*b*]furan-*N*^{*3*}-(4-nitrobenzoyl)amidinohydrazon-hydrochlorid **126**
(Z/E)-2-Benzoyl-5-brombenzo[*b*]furan-*N*^{*3*}-benzoylamidinohydrazon-hydrochlorid **127**
(Z/E)-2-Benzoyl-5,7-dibrombenzo[*b*]furan-*N*^{*3*}-benzoylamidinohydrazon-hydrochlorid **128**

Die Erfindung betrifft ferner ein Verfahren zur Herstellung der erfindungsgemäßen Amidinohydrazone der allgemeinen Formel I, das dadurch gekennzeichnet ist, daß man von Benzo[*b*]furan abgeleitete Ketone der allgemeinen Formel II, 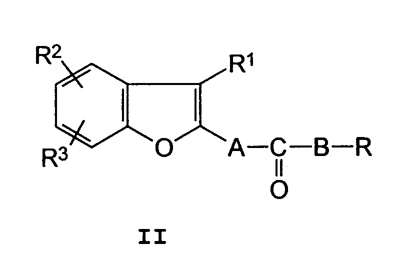 in der die Reste R, R¹, R² und R³ die oben angegebene Bedeutung haben,
mit einem Aminoguanidin der allgemeinen Formel III, 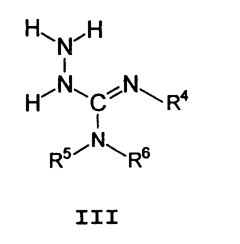 in der die Reste R⁴, R⁵ und R⁶ die oben angegebene Bedeutung haben, und wobei das Aminoguanidin gegebenenfalls als Salz mit einer anorganischen oder organischen Säure vorliegt,
durch Erhitzen in einem kurzkettigen Alkanol in Gegenwart von Mineral- bzw. Sulfonsäuren in an sich bekannter Weise kondensiert
und gegebenenfalls aus den so erhaltenen Verbindungen die Base freisetzt und diese Base
entweder mit einer oder mehreren physiologisch vertretbaren Säuren wie Mineralsäuren, linearen oder verzweigten Alkan- oder Alkensäuren oder Alkansulfonsäuren mit bis zu 6 C-Atomen oder Arencarbonsäuren, wobei die organischen Säuren gegebenenfalls mit Halogen-, Amino-, Dialkylamino- (mit bis zu 6-C-Atomen), Hydroxy- und Carboxy-Resten substituiert sind, umsetzt und in das physiologisch annehmbare Salz überführt,
oder in einem geeigneten Lösemittel, beispielsweise Pyridin, in Gegenwart von Hilfsbasen mit Acylhalogeniden der Formeln 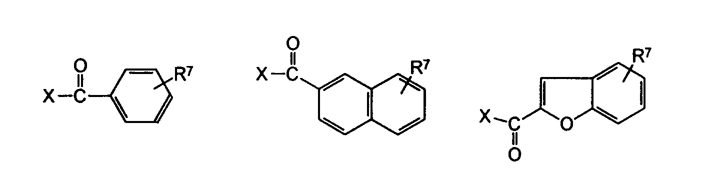 in denen R⁷ die in oben genannte Bedeutung hat und X ein Halogenatom bedeutet, umsetzt und die erhaltenen N-Acylderivate mit einer oder mehreren physiologisch vertretbaren Säuren wie Mineralsäuren, linearen oder verzweigten Alkan- oder Alkensäuren oder Alkansulfonsäuren mit bis zu 6 C-Atomen oder Arencarbonsäuren, wobei die organischen Säuren gegebenenfalls mit Halogen-, Amino-, Dialkylamino- (mit bis zu 6-C-Atomen), Hydroxy- und Carboxy-Resten substituiert sind, umsetzt und in das physiologisch annehmbare Salz überführt.

Schema A stellt eine Übersicht zur Darstellung der erfindungsgemäßen Amidinohydrazone dar, wobei nachfolgend aufgeführte substituierte Ketone wie folgt dargestellt wurden:
**2-Benzoylbenzo[*****b*****]furan:** Rap, E.: Gazz. Chim. Ital. **25**, 285 (1895); Schraufstätter, E.; Deutsch, D.: Z. Naturforsch. **46**, 276 (1949); Sen, A.B.; Saxena, M.S.: J. Indian Chem. Soc. **34**, 136 (1958); Ghelardoni, M.; Pestellini, V.; Musante, C.: Gazz. Chim. Ital. **99**, 1273 (1969);
**2-(4-Methoxybenzoyl)benzo[*****b*****]furan** und **2-(4-Methylbenzoyl)benzo[*****b*****]furan:** Buu Hoï, N.P.; Bisagni, E.; Royer, R.: J. Chem. Soc. **1957**, 625;
**2-(4-Chlorbenzoyl)benzo[*b*]furan** und **2-(4-Brombenzoyl)benzo[*****b*****]furan:** ibid., Ghelardoni, M.; Pestellini, V.; Musante, C.: Gazz. Chim. Ital. **99**, 1273 (1969);
**2-(4-Nitrobenzoyl)benzo[*****b*****]furan:** Ghelardoni, M.; Fedi, M.; Russo, F.: Ann. di Chim. **52**, 29 (1962);
**5-Brom-2-(4-brombenzoyl)benzo[*****b*****]furan:** Schraufstätter, E.; Deutsch, D.: Z. Naturforsch. **46**, 276 (1949);
**2-(3-Dimethylaminopropionyl)benzo[b]furan:** Knott, E.B.: J. chem. Soc. **1947**, 1190.

### Schema A

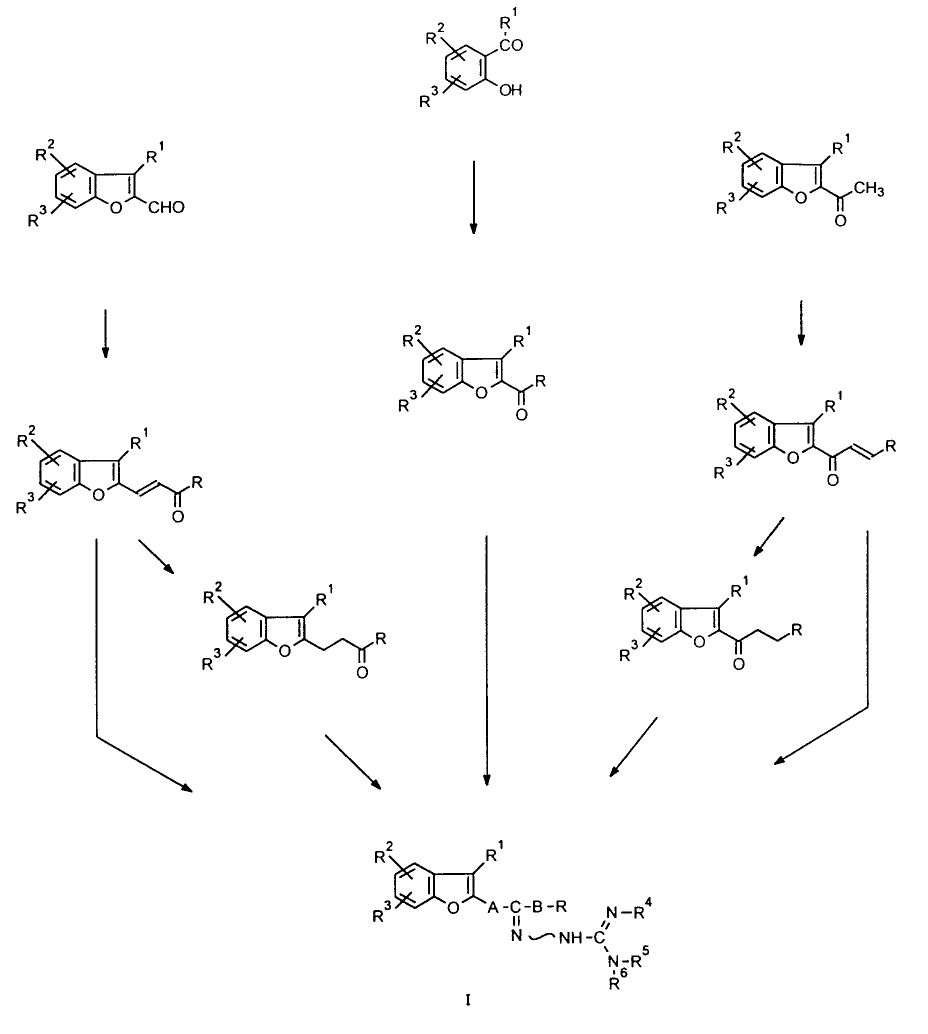

Im Schema A haben R, R¹, R², R³, R⁴, R⁵, R⁶, A und B die oben genannten Bedeutungen.

Die nach der Kondensation anfallenden Gemische der Isomeren werden durch dem Fachmann bekannte Methoden, wie beispielsweise der fraktionierten Kristallisation in die reinen (Z)- bzw. (E)-Isomere der Verbindungen der allgemeinen Formel I aufgetrennt.

Die bei der Kondensation anfallenden Salze werden beispielsweise mittels Alkalihydroxidlösungen in die zugrundeliegenden Basen überführt und diese mit entsprechenden Säuren zu physiologisch und/oder pharmazeutisch besser geeigneten Salzen der allgemeinen Formel I umgesetzt. Die Freisetzung der Basen kann gegebenenfalls auch in einem Lösungsmittel wie beispielsweise Pyridin in Gegenwart von Hilfsbasen durchgeführt werden.

Übliche physiologisch verträgliche anorganische und organische Säuren sind beispielsweise Salzsäure, Bromwasserstoffsäure, Phosphorsäure, Schwefelsäure, Oxalsäure, Maleinsäure, Fumarsäure, Milchsäure, Essigsäure, Weinsäure, Äpfelsäure, Citronensäure, Salicylsäure, Adipinsäure und Benzoesäure. Weitere verwendbare Säuren sind beispielsweise in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977) beschrieben.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol, n-Propanol oder Isopropanol oder einem niederen Keton wie Aceton, Methylethylketon oder Methyl-isobutylketon oder einem Ether wie Diethylether, Tetrahydrofuran oder Dioxan, erhalten. Zur besseren Kristallabscheidung können auch Mischungen der genannten Lösungsmittel verwendet werden. Darüber hinaus können physiologisch verträgliche wäßrige Lösungen von Säureadditionssalzen der Verbindung der Formel I in einer wäßrigen Säurelösung hergestellt werden.

Die Säureadditionssalze der Verbindungen der allgemeinen Formel I können in an sich bekannter Weise, z. B. mit Alkalien oder Ionenaustauschern, in die freie Base überführt werden. Von der freien Base lassen sich durch Umsetzung mit anorganischen oder organischen Säuren, insbesondere solchen, die zur Bildung von therapeutisch verwendbaren Salzen geeignet sind, weitere Salze gewinnen. Diese oder auch andere Salze der neuen Verbindung, wie z. B. das Pikrat, können auch zur Reinigung der freien Base dienen, indem man die freie Base in ein Salz überführt, dieses abtrennt und aus dem Salz wiederum die Base freisetzt.

Gegenstand der vorliegenden Erfindung sind auch Arzneimittel zur oralen, rektalen, subcutanen, percutanen, topischen, transdermalen, dermalen, intravenösen oder intramuskulären Applikation, die neben üblichen Träger- und Verdünnungsmitteln mindestens eine Verbindung der allgemeinen Formel I oder deren Säureadditionssalz als Wirkstoff enthalten.

Daneben können in den erfindungsgemäßen Arzneimitteln weitere Wirkstoffe vorhanden sein, welche insbesondere aus den Antiarrhythmika der Klassen I, II, III und/oder IV ausgewählt sind. Beispiele für Antiarrhythmika der Klasse I sind Flecainid, Lidocain, Propafenon, Beispiele für Klasse II-Antiarrhythmika sind Propranolol, Oxprenolol, Practolol, Acebutolol, Pindolol, Metindol, Nadolol, Labetalol, Bisoprolol, Tolamolol, Formoterol, Celiprolol, Bunitrolol, Atenolol, Metoprolol. Klasse III-Antiarrhythmika können beispielsweise sein Amiodaron, Sotalol, als Klasse IV-Antiarrhythmika seien Verapamil oder Diltiazem beispielhaft genannt.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Zubereitungen zur percutanen, topischen, dermalen oder transdermalen Applikation lassen sich durch Mischen der erfindungsgemäßen Verbindungen mit dem Fachmann bekannten Träger- und Hilfsstoffen nach dem Fachmann bekannten Verfahren herstellen.

Geeignete Suppositorien lassen sich beispielsweise durch Vermischen mit dafür vorgesehenen Trägermitteln wie Neutralfetten oder Polyäthylenglykol bzw. deren Derivaten herstellen.

Die erfindungsgemäßen Amidinohydrazone sind antiarrhythmisch wirksam, wobei ihre Wirkung in einer Verlängerung der kardialen Aktionspotentialdauer beruht, die mit einer Erhöhung der Refraktärperiode einhergeht. Die Verbindungen sind daher den Klasse III-Antiarrhythmika zuzuordnen und können insbesondere zur Behandlung und Prophylaxe ventrikulärer und supraventrikulärer Arrhythmien, die auf einem Reentry-Mechanismus beruhen und insbesondere bei chronischen Tachyarrhythmien eingesetzt werden.

Die kardioaktive Wirksamkeit der erfindungsgemäßen Substanzen besteht in einer Verlängerung der Repolarisationsphase (d. h. der Aktionspotentialdauer, im EKG der QT-Zeit), und somit einer Klasse III-Aktivität entsprechend der Einteilung der Antiarrhythmika nach Vaughan Williams. Antiarrhythmika der Klasse III sind in besonderer Weise zur Behandlung lebensbedrohender Herzrhythmusstörungen geeignet, da sie weder die Erregbarkeit der Herzzellen noch die Reizleitung im Herzen beeinflussen. Durch die Beeinflussung der repolarisierenden Kalium-Kanäle führen Klasse III-Antiarrhythmika zu einer Verlängerung der Aktionspotentialdauer und erhöhen so die Refraktärität des Myokards. Die Verlängerung der Refraktärzeit unter dem Einfluß dieser Substanzen geht mit einer Reduktion der Häufigkeit der gefährlichen sogenannten Reentry-Arrhythmien einher. Reentry-Arrhythmien werden als Hauptursache des Kammerflimmerns angesehen, welches die den plötzlichen Herztod verursachende Rhythmusstörung darstellt.

Antiarrhythmische Eigenschaften können in verschiedenen experimentellen Modellen *in vitro* und *in vivo* untersucht werden. Geeignet dafür sind elektrophysiologische Untersuchungen an isolierten Myokardpräparaten - z. B. Purkinje Faser, Papillarmuskel, Vorhof- und Kammergewebe - von Meerschweinchen, Ratten, Kaninchen oder Hund. Dabei werden die Wirkungen der erfindungsgemäßen Verbindungen auf das Ruhe- und Aktionspotential im Vergleich zu Plazebo und einer Vergleichssubstanz untersucht. Bei *in vivo* Prüfungen werden oftmals Hunde oder Schweine eingesetzt, da diese zu den am besten untersuchten Versuchstieren zählen und daher umfangreiche Vergleichsdaten zur Verfügung stehen. Die zu prüfenden Verbindungen werden dann bei solchen Tieren appliziert, die infolge eines vorher durch geeignete Methoden induzierten Myokardinfarkts die zu behandelnden Rhythmusstörungen aufweisen. Diese Untersuchungen können entweder unter Narkosebedingungen durchgeführt werden, wobei mittels programmierter Kammerstimulation die tachykarden Reentryarrhythmien ausgelöst werden oder in dem Versuchsmodell des plötzlichen Herztodes am wachen Hund, in dem die Tiere während einer physischen Belastung zusätzlich einer akute Ischämie unterzogen werden, wobei nicht behandelte Tiere in über 90% der Fälle Kammerflimmern entwickeln.

Die antiarrhythmische Wirksamkeit der erfindungsgemäßen Amidinohydrazone vom Benzo[*b*]furan abgeleiteter Ketone belegen die nachfolgend aufgeführten Versuchsergebnisse.

Da bei den zu untersuchenden Verbindungen ein breites Spektrum der gewünschten elektrophysiologischen Eigenschaften sowie der Zeitverlauf der erwünschten Wirkungen geprüft werden sollten, wurde ein spezifisches Versuchsmodell entwickelt.

Als Versuchstiere dienten Meerschweinchen mit einem Körpergewicht von ca. 300 g. Pro Substanz wurden 5 Tiere eingesetzt. Die Tiere erhielten die erfindungsgemäßen Substanzen in äquimolaren Dosierungen und als Referenzsubstanzen 80 mg/kg Amiodaron und Plazebo als intraperitoneale Injektion 1x täglich über eine Dauer von insgesamt 7 Tagen injiziert. An den Tagen 3, 5 und 7 wurden EKG Messungen vorgenommen, um den Effekt der verabreichten Substanzen auf die Herzfrequenz (das RR-Intervall) und speziell auf das QT Intervall im EKG (als Maß für die Erregungsausbreitung und die Repolarisation) zu prüfen. An Tag 8 wurden die Tiere 24 Stunden nach der letzten Substanzinjektion getötet und aus den Herzen der Papillarmuskel für die elektrophysiologischen Untersuchungen entnommen.

Bei diesen Untersuchungen wurde bewußt Amiodaron als Vergleichssubstanz gewählt, da dieses als effektivste antiarrhythmisch wirksame Substanz anerkannt ist, die zudem keine sogenannte *reverse use dependence* besitzt, d.h. bei hohen Herzfrequenzen (i.e. kurzer Stimulationszykluslänge) nicht ihre verlängernde Wirkung auf das Aktionspotential und die Refraktärzeiten verliert.

**Tabelle 1**

| **Qualitative EKG-Effekte** | | |
|---|---|---|
| **Verbindung** | **RR Intervall** | **QT Intervall (frequenzkorrigiert)** |
| **Amiodaron** | 0 | + |
| **101** | 0 | + |
| **106** | + | + |
| **126** | + | + |
| **127** | 0 | 0 |
| **128** | 0 | 0 |
| +: signifikante Verlängerung (p < 0.05 im Vergleich zu Plazebo) 0: kein Effekt im Vergleich zu Plazebo | | |

**Tabelle 2**

| **Qualitative Wirkungen auf das Aktionspotential** | | | | | |
|---|---|---|---|---|---|
| **Verbindung** | **MDP** | **APA** | 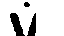 _{**max**} | **APD**_{**50**} | **APD**_{**90**} |
| **Amiodaron** | 0 | 0 | 0 | + | + |
| **101** | 0 | 0 | 0 | + | + |
| **106** | 0 | 0 | 0 | + | + |
| **126** | 0 | 0 | 0 | + | + |
| **127** | 0 | 0 | 0 | + | + |
| **128** | 0 | 0 | 0 | 0 | + |
| +: signifikante Verlangerung (p < 0.05 im Vergleich zu Plazebo) 0: kein Effekt im Vergleich zu Plazebo APA = Amplitude des Aktionspotentials, APD₅₀ = Aktionspotentialdauer bei 50% Repolarisation, APD₉₀ = Aktionspotentialdauer bei 90% Repolarisation, MDP = maximales diastolisches Potential, 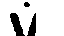 ₘₐₓ = maximale Aufstrichsgeschwindigkeit des Aktionspotentials | | | | | |

Die Wirkungen der erfindungsgemäßen Verbindungen auf die relevanten Parameter des EKG und des Aktionspotentials sind in den Tabellen 1 und 2 qualitativ zusammengefaßt und jeweils den mit Amiodaron erhaltenen Ergebnissen gegenübergestellt. Hierbei ist besonders hervorzuheben, daß die neuen zu prüfenden Verbindungen, wie gewünscht, mit signifikanten Verlängerungen der Dauer des Aktionspotentials (APD₅₀ und APD₉₀) und des QT-Intervalls die gesuchten Charakteristiken zeigen. Hinsichtlich der anderen Parameter des Aktionspotentials, dem maximalen diastolischen Potential (MDP), das dem Ruhepotential entspricht und für die Erregbarkeit der Herzzellen verantwortlich ist, der Aufstrichsgeschwindigkeit ( 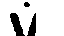 ₘₐₓ) des Aktionspotentials bei der elektrischen Erregung und der Aktionspotentialamplitude (APA), die für die Erregungsausbreitung und die Leitungsgeschwindigkeit der Erregung von entscheidender Bedeutung sind, haben die neuen Verbindungen, wiederum wie gewünscht und ähnlich dem Amiodaron, keinen Einfluß.

Eine der entscheidenden Vorzüge von Amiodaron gegenüber Sotalol und anderen, noch in der Entwicklung befindlichen Klasse III-Antiarrhythmika ist das Fehlen der sogenannten *reverse use dependence* bei chronischer Applikation von Amiodaron. Damit wird die Eigenschaft bezeichnet, daß die erwünschte Wirkung, nämlich die Aktionspotentialverlängerung und Refraktärzeiterhöhung, bei hohen Herzfrequenzen, bei denen im vorgeschädigten Herzen besonders die Gefahr lebensbedrohlicher Reentry-Tachykardien droht, wieder abnimmt. Bei der Forschung nach neuen Verbindungen ist daher die Forderung zu stellen, solche Verbindungen zu finden, die keine *reverse use dependence* aufweisen. Die erfindungsgemäßen Verbindungen erfüllen diese Anforderung.

Tabelle 3 gibt Aufschluß über die quantitativen Veränderungen einzelner elektrophysiologischer Parameter nach chronischer Verabreichung eines Vertreters der erfindungsgemäßen Verbindungen respektive nach chronischer Verabreichung von Amiodaron im Vergleich zu Plazebo. Es werden speziell die prozentualen Veränderungen der Refraktärzeiten (ERP) des Vorhof- und des Ventrikelmyokards und die Veränderungen der Aktionspotentialdauer (APD) des Papillarmuskels bei langsamer (1000 ms) und schneller (300 ms) Stimulationsfrequenz dargestellt.

**Tabelle 3**

| **Quantitative Veränderungen elektrophysiologischer Parameter** | | |
|---|---|---|
| | **Amiodaron** | **126** |
| ERP_{Papillarmuskel} | + 18,7 % * | + 19,6 % * |
| ERP_{linker Vorhof} | + 21,3 % * | + 24,0 % * |
| APD₅₀ (1000 ms) | + 11,5 % * | + 23,8 % * |
| APD₉₀ (1000 ms) | + 11,6 % * | + 21,8 % * |
| APD₅₀ (300 ms) | + 19,7 % * | + 28,4 % * |
| APD₉₀ (300 ms) | + 15,6 % * | + 19,3 % * |

| | | |
|---|---|---|
| * signifikant gegenüber Plazebo mit p < 0.05 | | |
| ERP = effektive Refraktärperiode (gemessen am Papillarmuskel resp. im linken Vorhof bei der Stimulationszykluslänge von 300 ms) APD₅₀ (1000 ms) = APD₅₀ bei der Stimulationszykluslänge von 1000 ms APD₉₀ (1000 ms) = APD₉₀ bei der Stimulationszykluslänge von 1000 ms APD₅₀ (300 ms) = APD₅₀ bei der Stimulationszykluslänge von 300 ms APD₉₀ (300 ms) = APD₉₀ bei der Stimulationszykluslänge von 300 ms | | |

Die Ergebnisse belegen, daß die erfindungsgemäßen Verbindungen eine ausgeprägte Klasse III-Aktivität entfalten, die keiner *reverse use dependence* unterliegt, d.h., daß die antiarrhythmische, repolarisationsverlängernde Wirkung nicht bei hohen Herzfrequenzen und tachykarden Zuständen, wo sie gerade benötigt werden, verloren geht. Die erfindungsgemäßen Amidinohydrazone entfalten somit Wirkungen, die denen des Amiodaron mindestens entsprechen wenn nicht sogar überlegen sind.

Folgende Beispiele erläutern die Erfindung:

### Beispiel 1

### 2-Benzoylbenzo[b]furan 1

24,4 g Salicylaldehyd und 300 ml Ethanol werden mit 11,6 g Kaliumhydroxid versetzt und die Suspension bei Raumtemperatur gerührt, bis alles Kaliumhydroxid aufgelöst ist. Dann werden der Lösung portionsweise 31 g Phenacylchlorid zugesetzt und der Ansatz unter Rühren 2 h am Rückflußkühler erhitzt. Die nach der Filtration beim Abkühlen auf Raumtemperatur abgeschiedenen Kristalle werden abgesaugt, mit Wasser gewaschen und aus Methanol umkristallisiert. Eine weitere Fraktion kann durch Einengen der Mutterlauge erhalten werden. Ausbeute: 70%, farblose Kristalle. Schmelzbereich: 84 - 86 °C

Analog werden die in den Tabellen 4 und 5 aufgeführten Verbindungen erhalten.

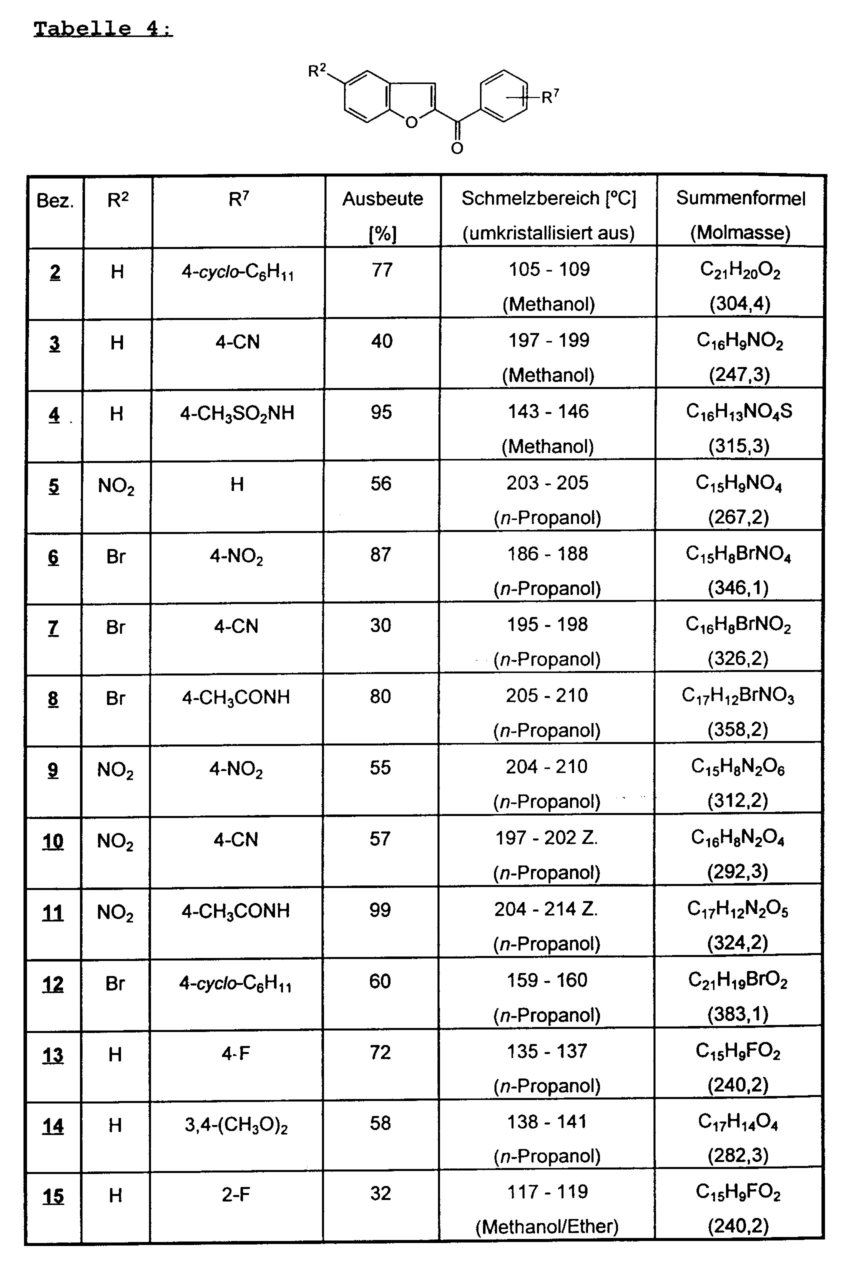

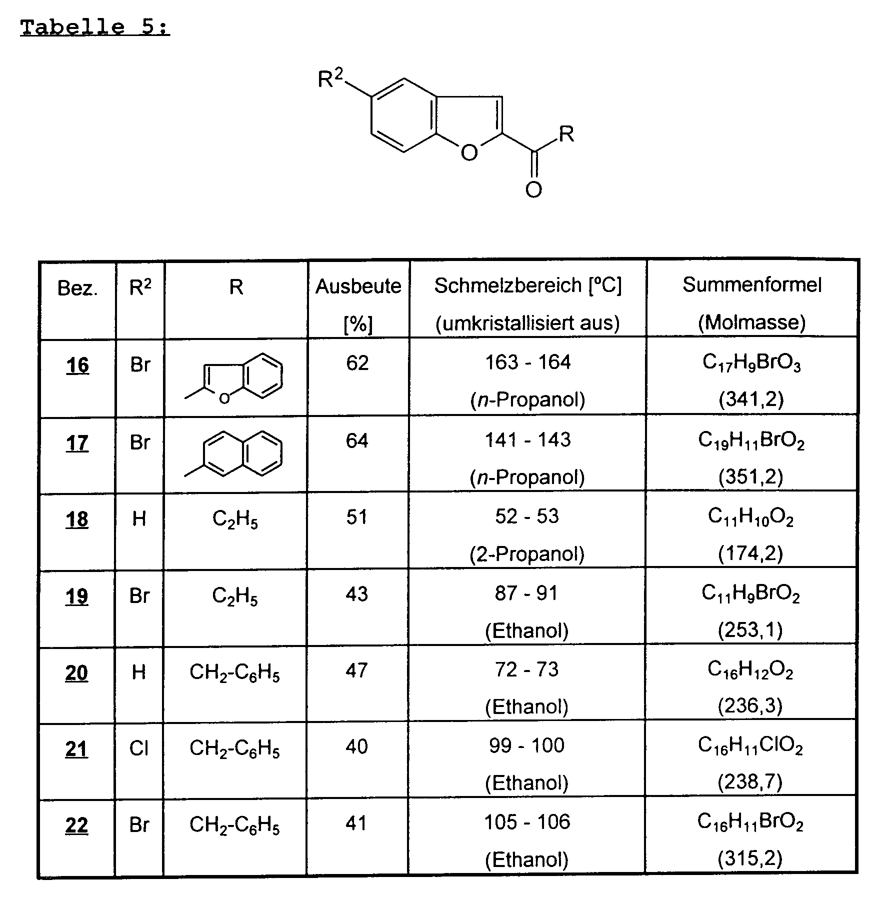

### Beispiel 2

### 1-(Benzo[b]furan-2-yl)-3-phenylprop-2-en-1-on 23

8 g 2-Acetylbenzo[*b*]furan und 6 g Benzaldehyd werden in 50 ml Methanol in Gegenwart von 3 ml konzentrierter Salzsäure zum Sieden erhitzt. Nach 10 h wird der Ansatz abgekühlt, die ausgefallene gelbe Kristallmasse abgesaugt und mit wenig kaltem Methanol nachgewaschen. Ausbeute: 60%, gelbe Kristalle Schmelzbereich: 109 - 111 °C.

In Analogie zu dieser Vorschrift wurden die in Tabelle 6 aufgeführten Verbindungen dargestellt. Meist sind die so erhaltenen Substanzen bereits rein genug, sie lassen sich erforderlichenfalls aus Methanol o.ä. umkristallisieren. Die Ausbeuten liegen zwischen 50% und 97% der Theorie. Beliebig substituierte Benzo[*b*]furan-2-carboxaldehyde und Acetophenone lassen sich auf analogem Wege in ebenfalls guten Ausbeuten zur Kondensation bringen.

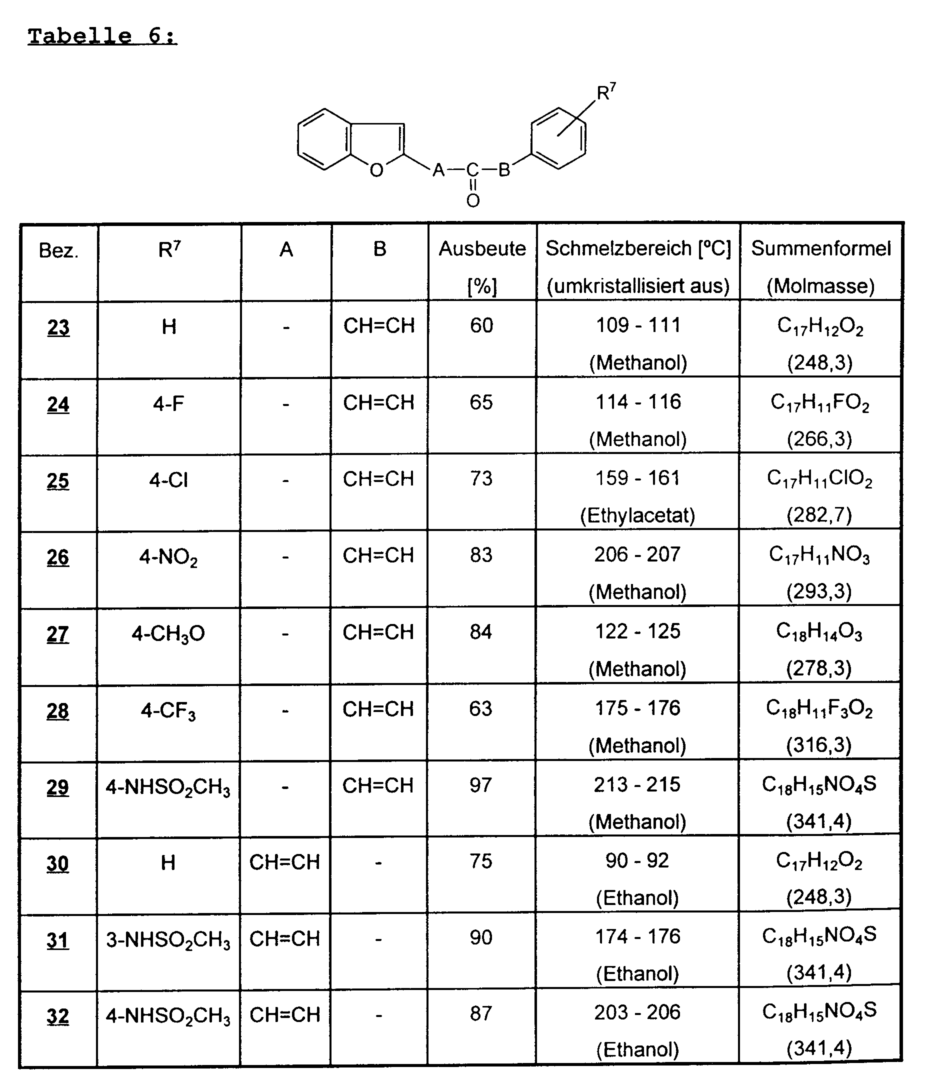

### Beispiel 3

### 1-(Benzo[b]furan-2-yl)-3-phenylpropan-1-on 33

Eine 10%ige methanolische Lösung der Ethenylverbindung **23** wird mit 10% Palladium an Aktivkohle unter leichtem Überdruck bis zur theoretischen Wasserstoffaufnahme hydriert, filtriert und die Lösung hernach im Vakuum eingedampft. Die so erhaltene Verbindung wird aus 2-Propanol umkristallisiert. Ausbeute: 97%, weiße Kristalle Schmelzbereich: 61 - 63 °C.

Gemäß dieser Vorschrift wurden die in Tabelle 7 aufgeführten Verbindungen dargestellt. Meist sind die so erhaltenen Substanzen bereits rein genug, sie lassen sich erforderlichenfalls aus Methanol o.ä. umkristallisieren. Die Ausbeuten liegen zwischen 43% und 80% der Theorie.

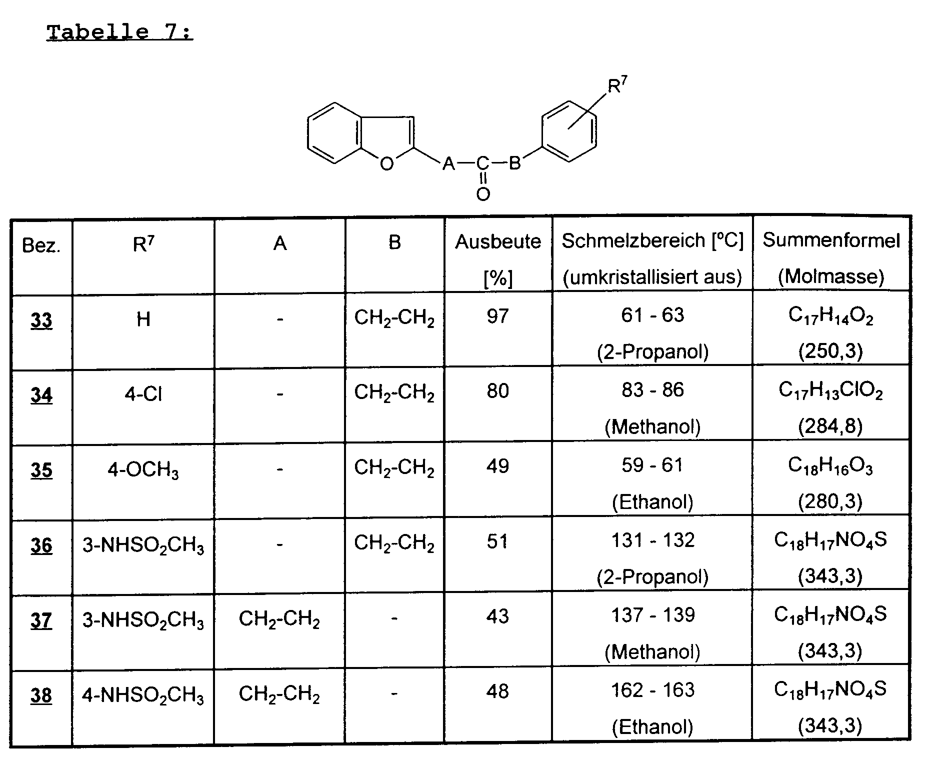

### Beispiel 4

### (E)- und (Z)-2-Benzoylbenzo[b]furanamidinohydrazon-hydrochlorid 39 und 40

7,7 g 2-Benzoylbenzo[*b*]furan und 5,1 g Aminoguanidin-hydrochlorid werden mit 40 ml Ethanol und 8 ml konzentrierter Salzsäure übergossen und der Ansatz 5 h unter Rühren am Rückflußkühler erhitzt. Anschließend wird die Lösung auf 5 °C abgekühlt und das Produkt zur Kristallisation gebracht. Es wird abgesaugt und diese erste Fraktion aus Ethanol umkristallisiert.
**(E)-2-Benzoylbenzo[*b*]furanamidinohydrazon-hydrochlorid 39**; Ausbeute: 41%. Farblose Kristalle (vgl. Tabelle 8).

Nach Abtrennung vorstehender Verbindung kristallisiert aus der Mutterlauge bei weiterem Aufbewahren im Kühlschrank eine zweite Fraktion, die abgesaugt und aus Ethanol umkristallisiert wird.
**(Z)-2-Benzoylbenzo[*****b*****]furanamidinohydrazon-hydrochlorid** **40**; Ausbeute: 29 %. Farblose Kristalle (vgl. Tabelle 8).

Analog werden die in den Tabellen 8 bis 16 aufgeführten Verbindungen erhalten, die in der Regel als Isomerengemische isoliert werden.

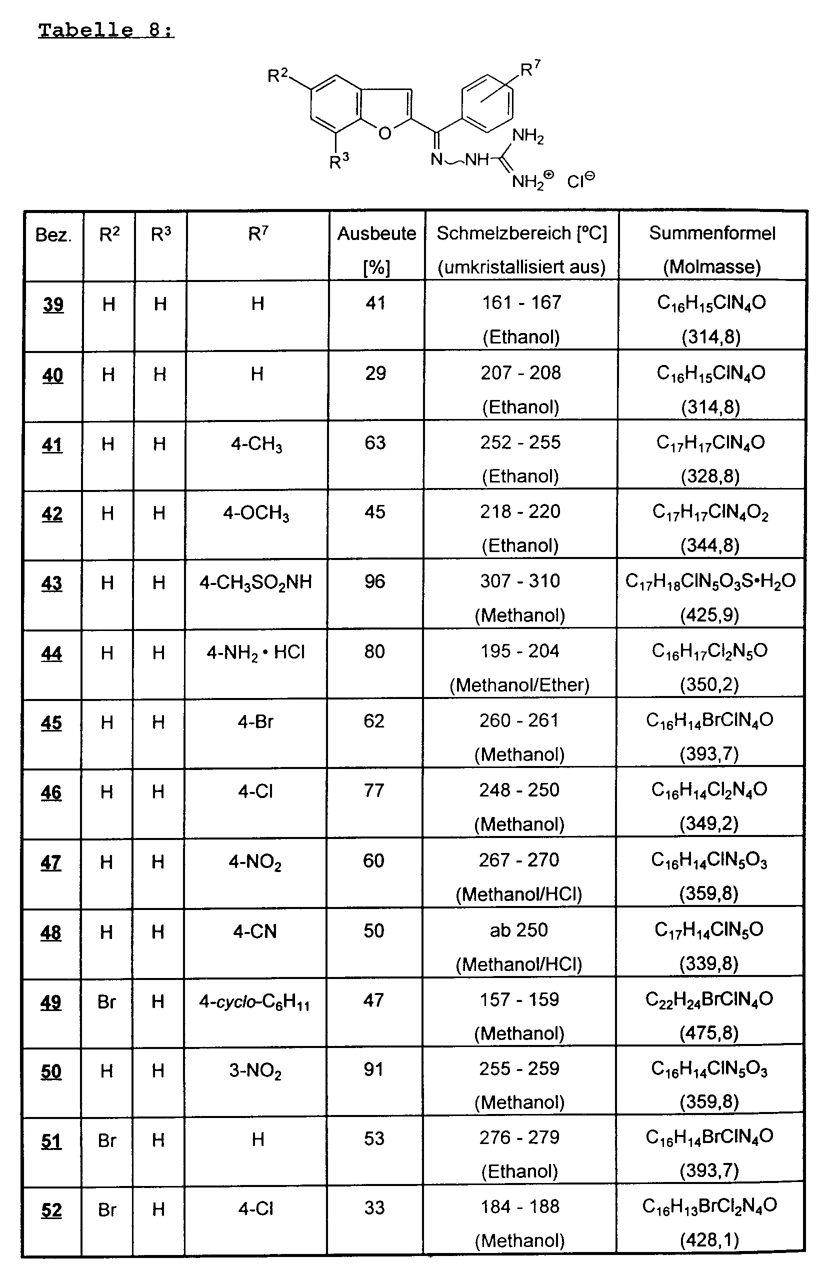

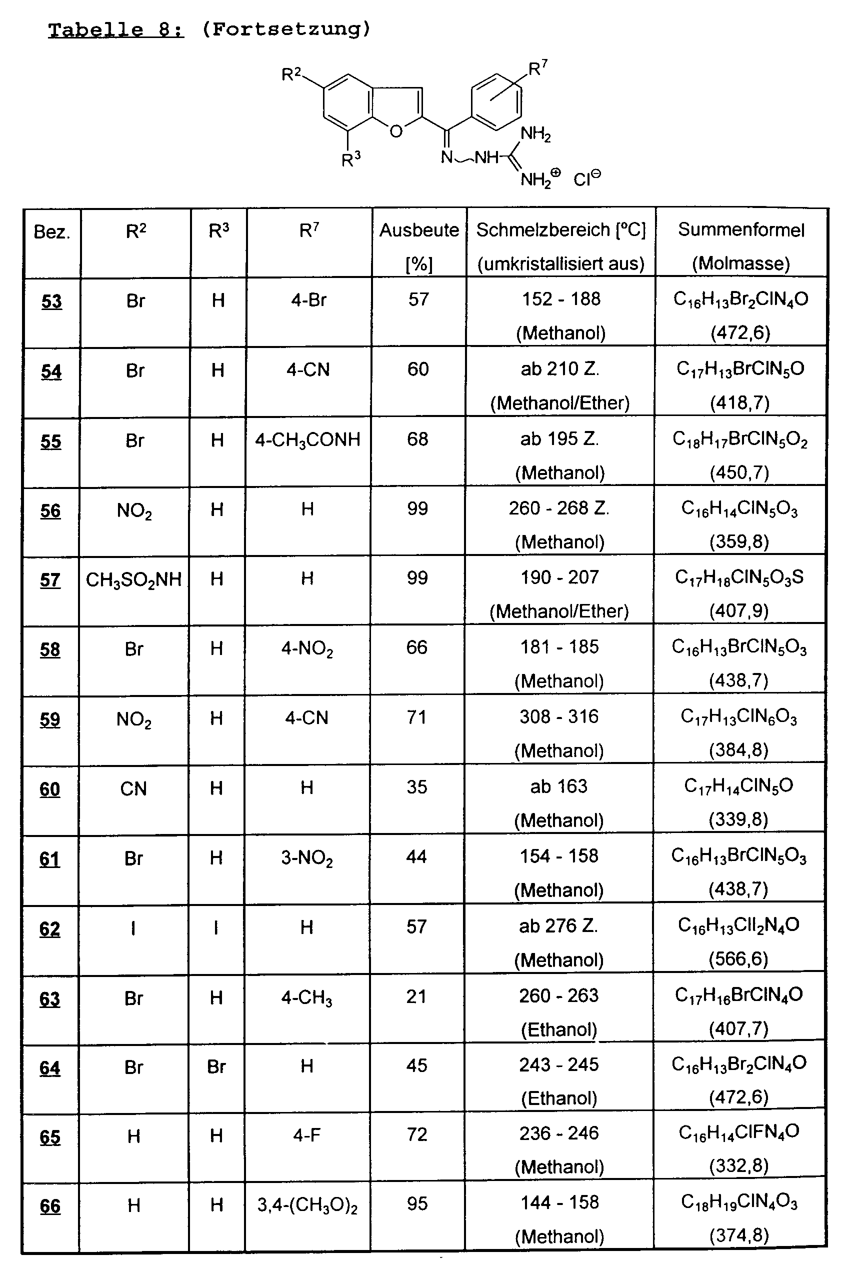

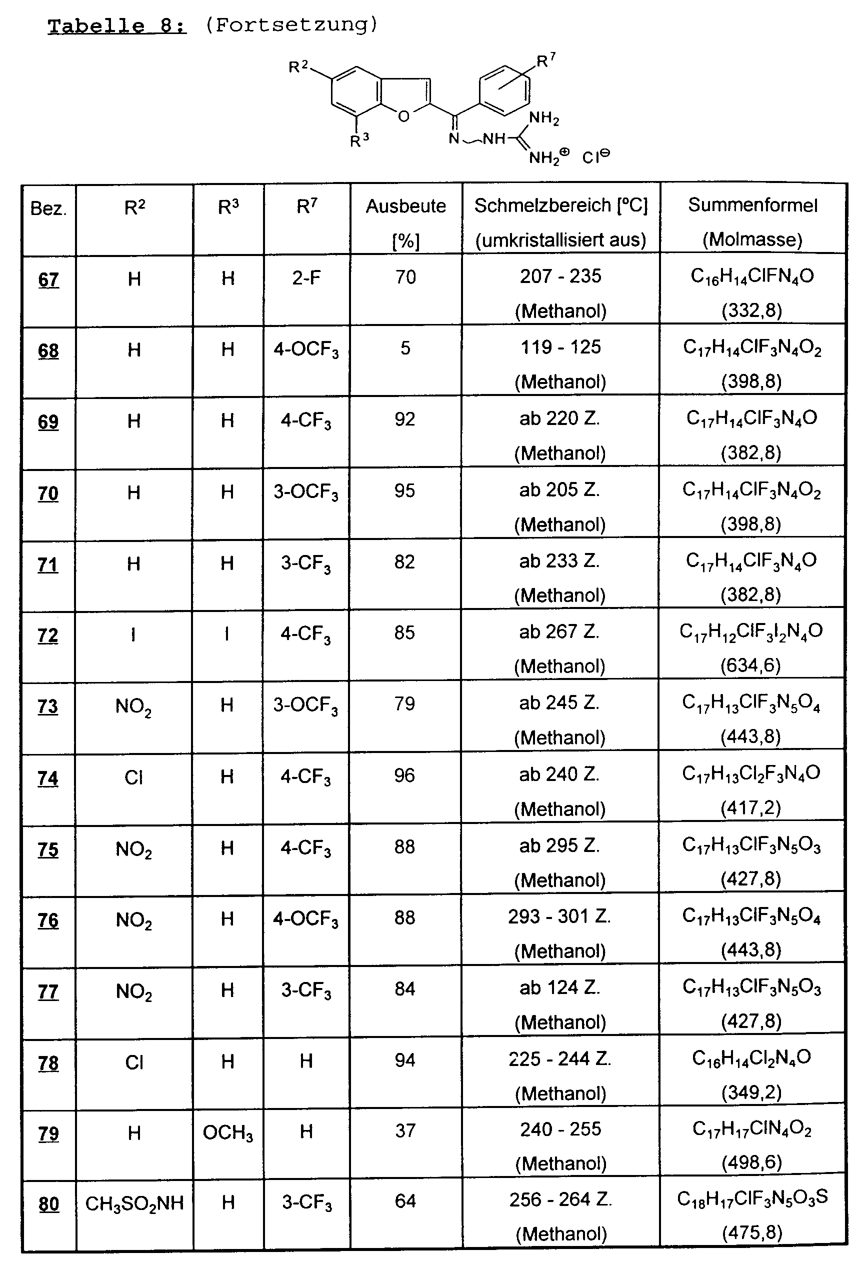

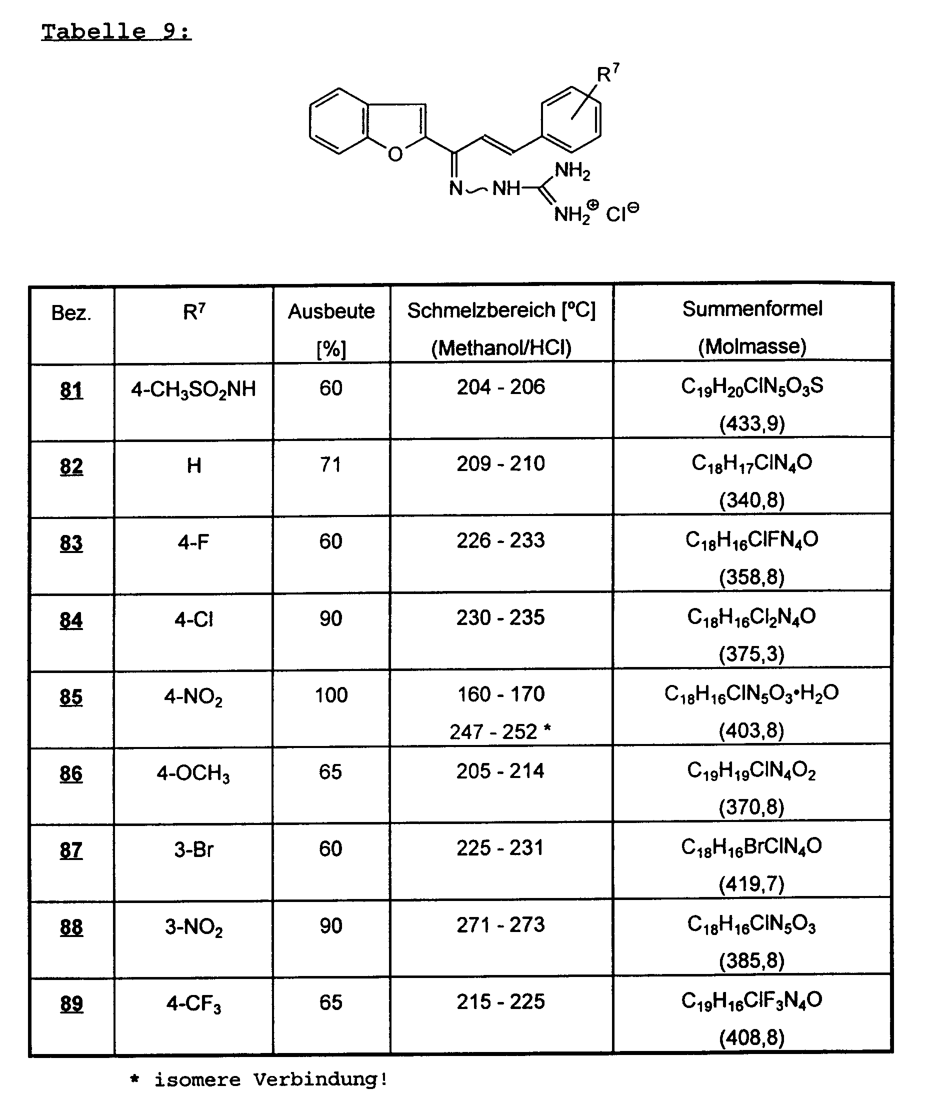

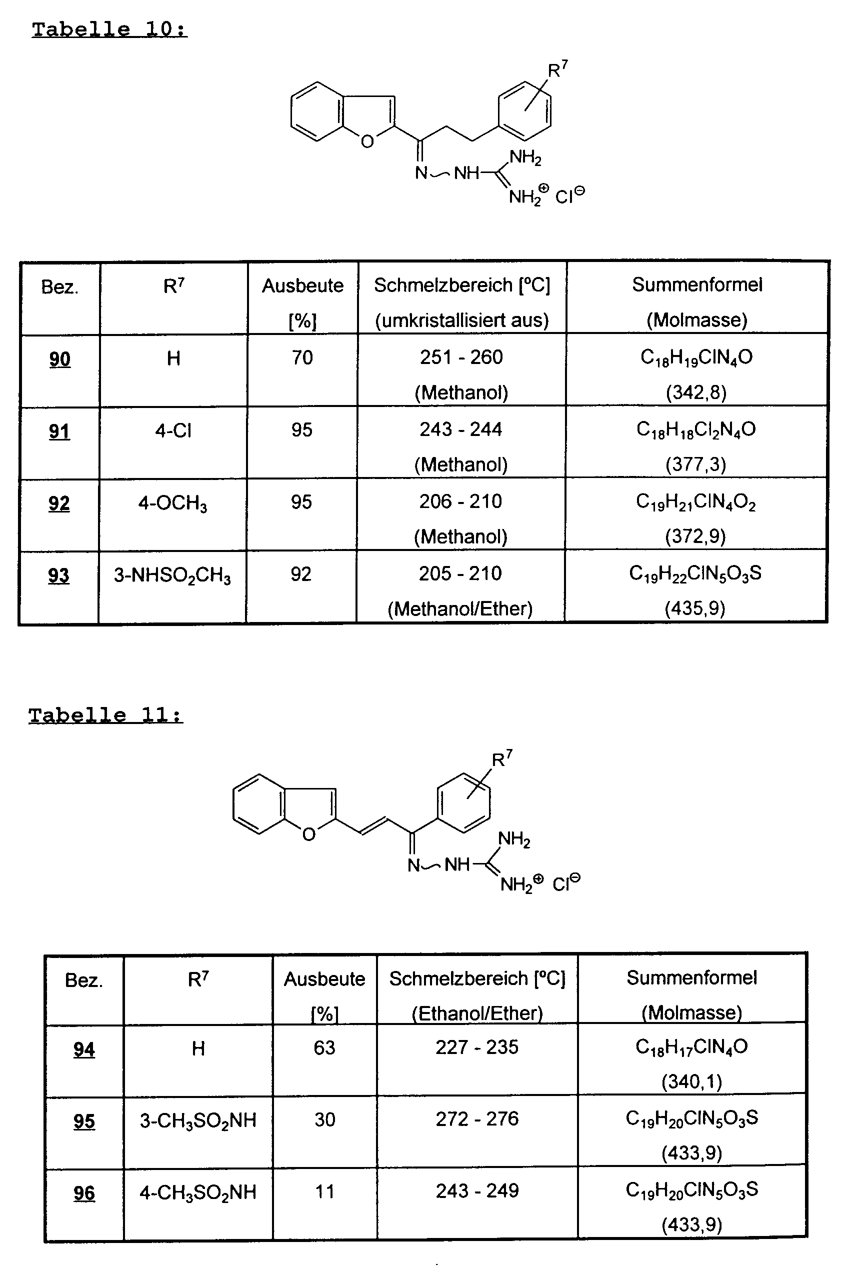

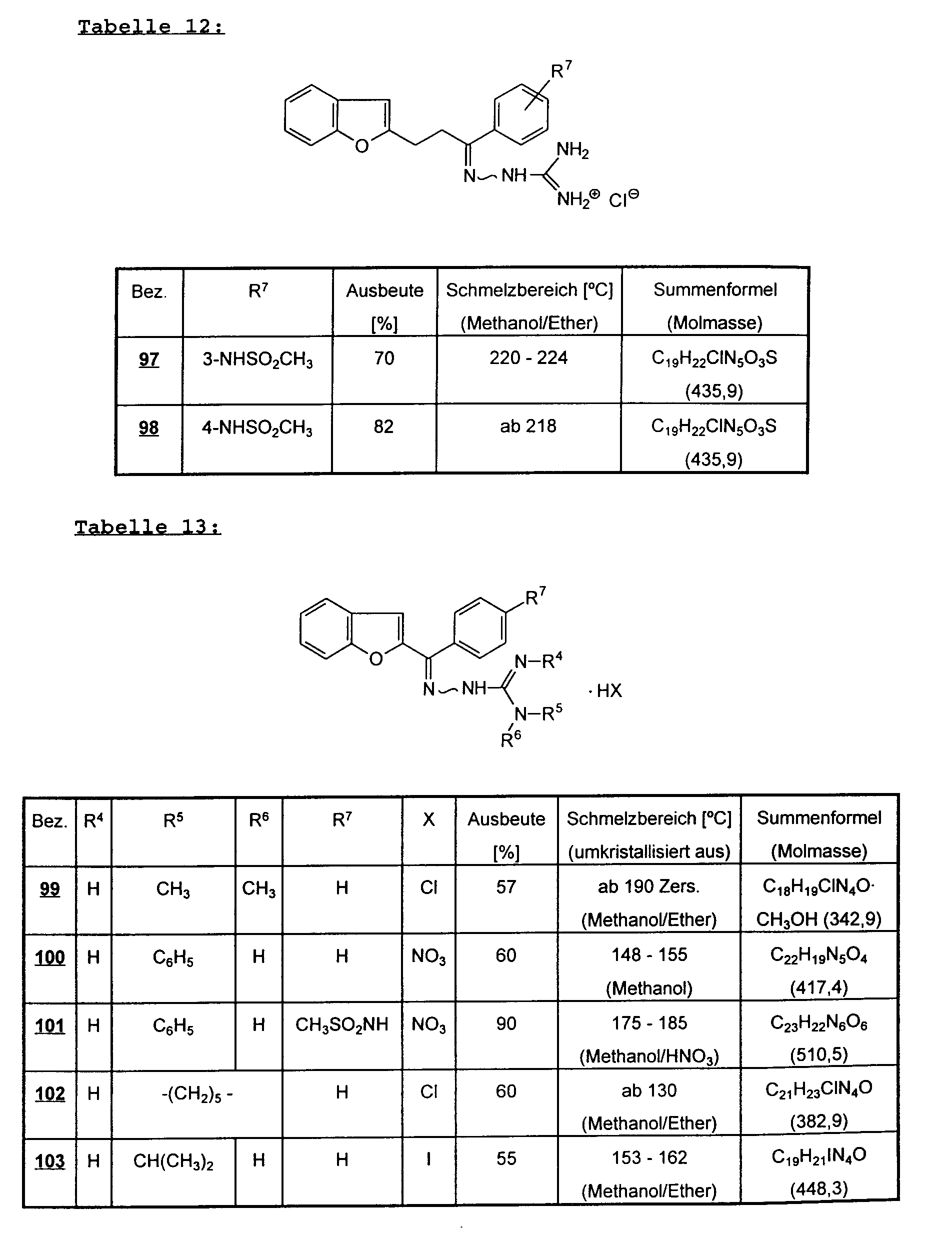

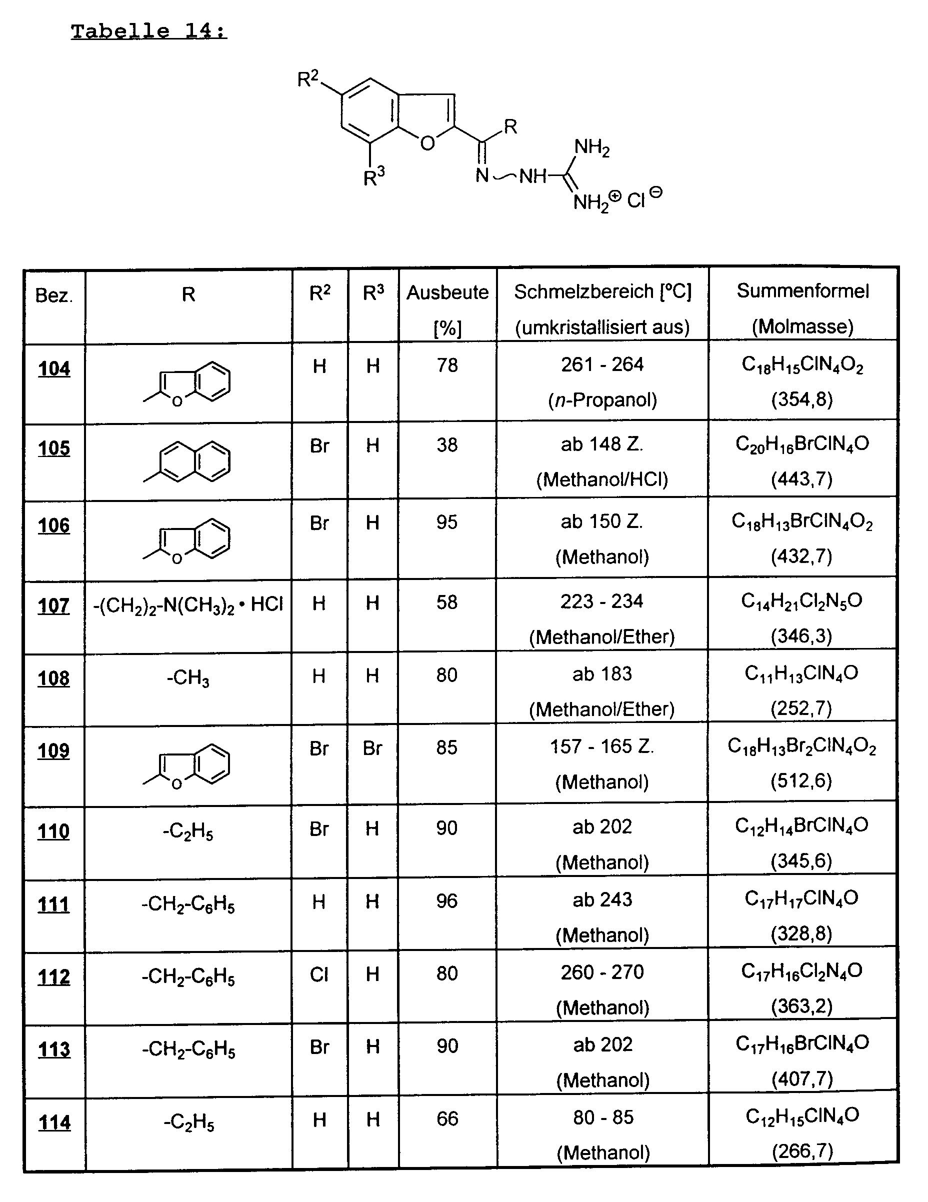

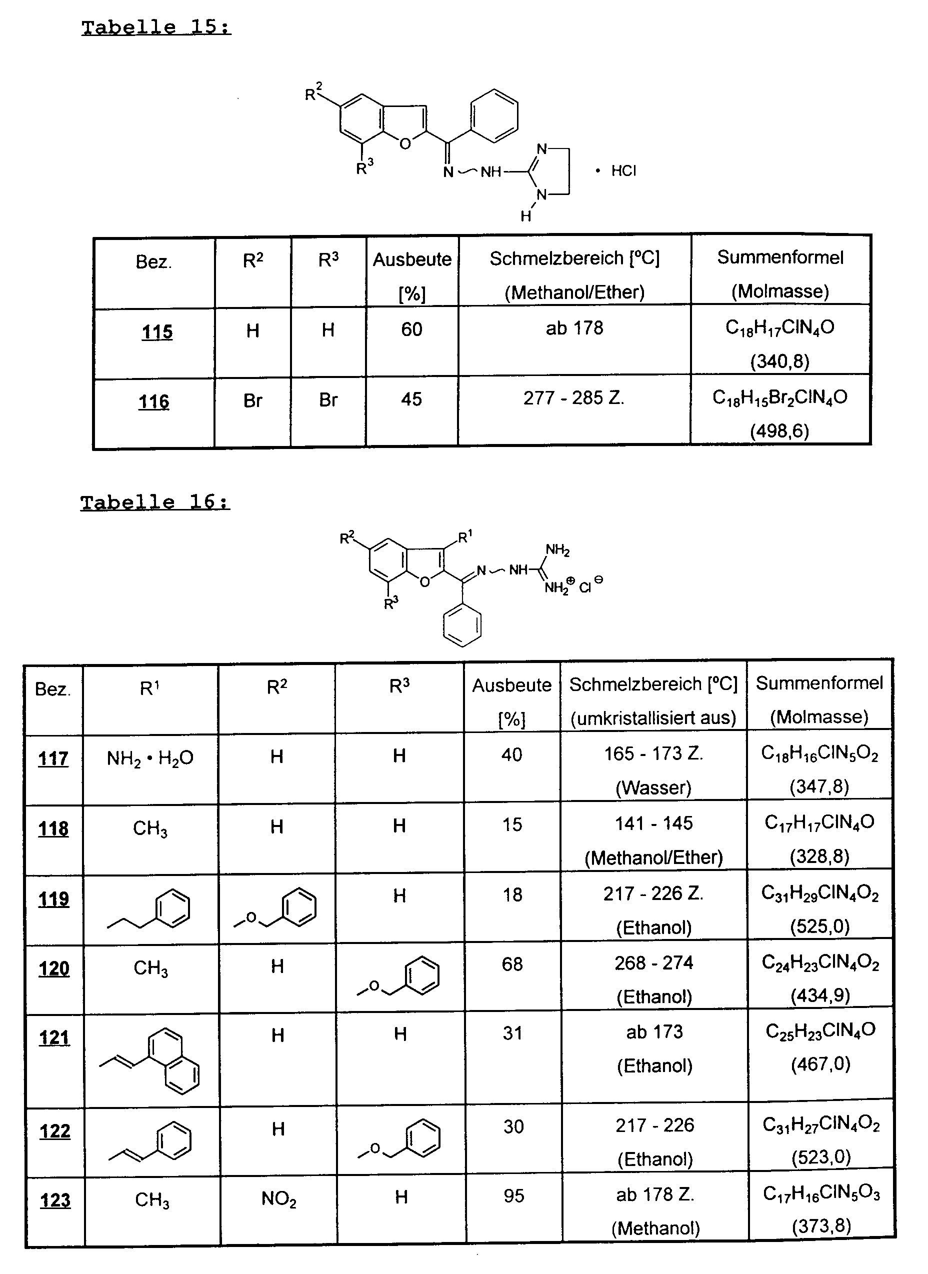

### Beispiel 5

### 2-Benzoylbenzo[b]furan-N³-benzoylamidinohydrazon-hydrochlorid 121

3,0 g (Z/E)-2-Benzoylbenzo[*b*]furanamidinohydrazon-hydrochlorid **39**/**40** werden unter leichtem Erwärmen in 300 ml Wasser gelöst und die Lösung nach dem Erkalten mit 5%iger Kaliumhydroxidlösung alkalisiert. Der abgeschiedene Niederschlag wird abgesaugt, mit Wasser gewaschen und getrocknet.
Zu einer eisgekühlten Lösung von 1,4 g 2-Benzoylbenzo[*b*]furanamidinohydrazon, 0,85 ml Diisopropyl-ethylamin und 1,2 g 4-(*N*,*N*-Dimethylamino)pyridin in 14 ml trockenem Pyridin werden 0,85 ml Benzoylchlorid, gelöst in 8 ml trockenem Dioxan, unter Rühren zugetropft. Die orangefarbene Lösung wird weitere 2 h gerührt. Der Ansatz wird anschließend in Eiswasser gegossen, der sich abscheidende gelbe Niederschlag abgesaugt, mit Wasser gewaschen und getrocknet. Das so gewonnene Kristallpulver wird mit 20 ml chlorwasserstoffgesättigtem Methanol behandelt, anschließend das Lösemittel im Vakuum gut abdestilliert und die Substanz durch Kodestillation mit trockenem Toluol im Hochvakuum getrocknet. Zur Reinigung wird die Substanz in Chloroform/Methanol gelöst und zur fraktionierten Fällung zunächst mit Ethylacetat und dann mit Ether bis zur starken Trübung versetzt. Der entstandene Niederschlag wird abgesaugt, mit wenig Ether gewaschen und getrocknet.

Analog werden die in Tabelle 17 aufgeführten Verbindungen erhalten.

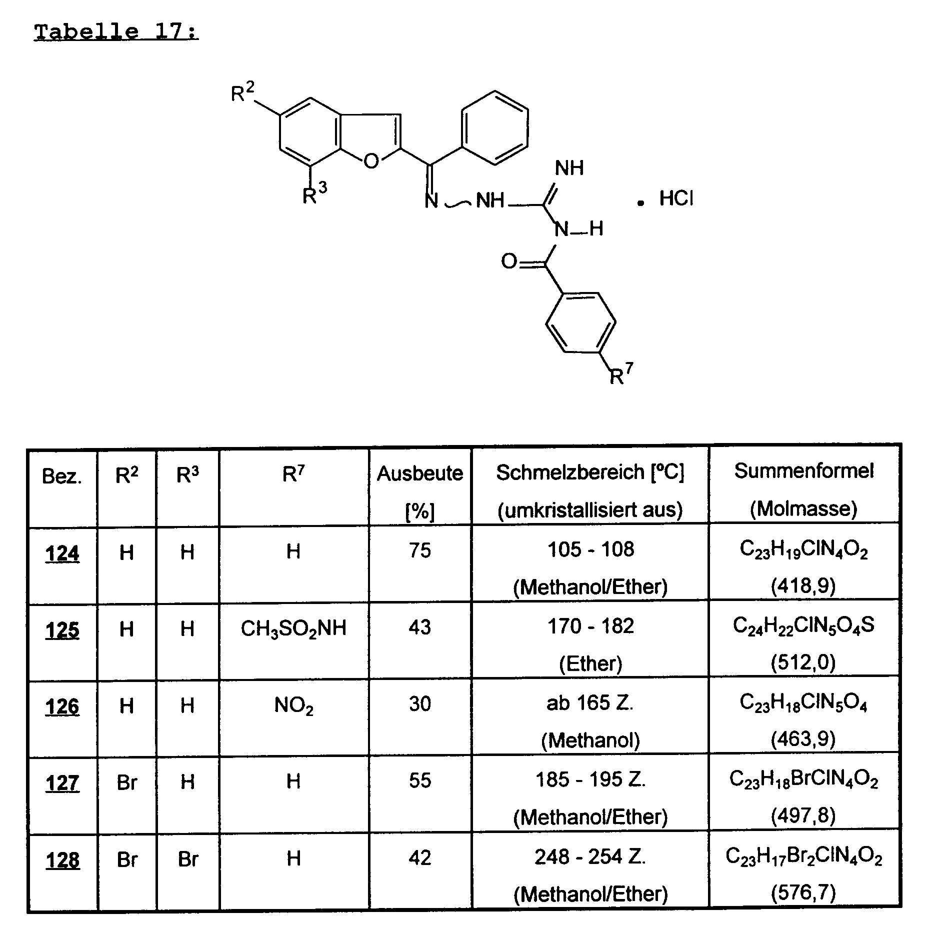

## Patentansprüche

1. Amidinohydrazone der allgemeinen Formel I, 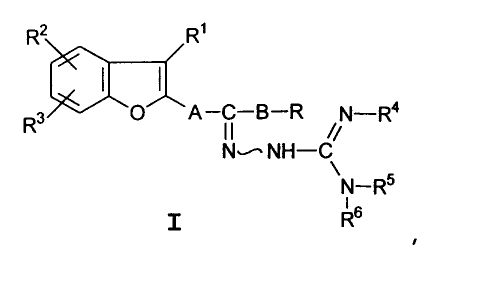 worin
R eine lineare oder verzweigte Alkyl- oder Dialkylaminoethylgruppe mit jeweils bis zu 6 C-Atomen oder einen der Reste 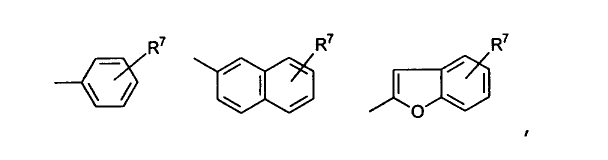 worin
R⁷ ein Wasserstoffatom, ein Halogenatom, eine lineare oder verzweigte Alkyl- oder Alkoxygruppe mit bis zu 6 C-Atomen, eine Aralkyl- oder Aralkoxygruppe mit bis zu 9 C-Atomen, eine Cyano-, eine Nitro-, eine Methansulfonamido-, eine Acetylamino-, eine Trifluormethyl-, eine Trifluormethoxy-, eine Amino- oder eine (1*H*-Imidazol-1-yl)-Gruppe ist,
darstellt,
A und B unabhängig voneinander die Bedeutung (CH₂)ₙ oder (CH=CH)ₘ haben, wobei n = 0, 1 oder 2 und m = 0 oder 1 ist,
R¹ ein Wasserstoffatom, ein Amino-, ein linearer oder verzweigter Alkylrest mit bis zu 6 C-Atomen, ein Aralkylrest mit bis zu 9 C-Atomen, ein Methansulfonamido-, ein Acetylamino-, ein Cyano-, ein (1*H*-Imidazol-1-yl)-Rest oder einer der Reste 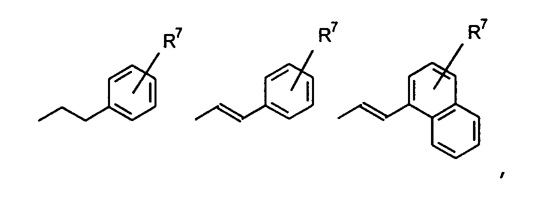 worin
R⁷ die oben angegebene Bedeutung hat, ist,
R² und R³ unabhängig voneinander ein Wasserstoffatom, eine lineare oder verzweigte Alkyl- oder Alkoxygruppe mit bis zu 6 C-Atomen, eine Aralkyl- oder Aralkoxygruppe mit bis zu 9 C-Atomen, ein Halogenatom, eine Cyano-, eine Nitro-, eine Methansulfonamido-, eine Acetylamino-, eine Trifluormethyl-, eine Trifluormethoxy-, eine Amino- oder eine (1*H*-Imidazol-1-yl)-Gruppe sind,
R⁴ ein Wasserstoffatom, eine lineare oder verzweigte Alkylgruppe mit bis zu 6 C-Atomen, eine Aralkylgruppe mit bis zu 9 C-Atomen oder einen der Reste 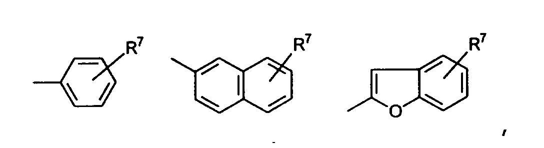 worin
R⁷ die oben angegebene Bedeutung hat, darstellt,
R⁵ und R⁶ unabhängig voneinander ein Wasserstoffatom, einen linearen oder verzweigten Alkyl-, Alkanoyl- oder Alkansulfonylrest mit jeweils bis zu 6 C-Atomen, einen Aralkylrest mit bis zu 9 C-Atomen, einen (4-Methylphenyl)sulfonyl-, einen Trifluoracetyl- oder einen der Reste 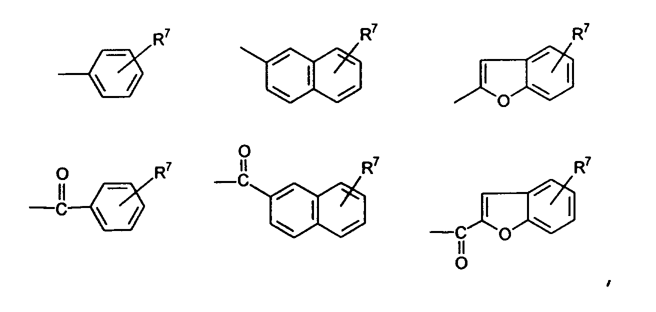 worin
R⁷ die oben angegebene Bedeutung hat, bedeuten,
oder R⁴ und R⁵ zusammen ein Ethylen- oder Propylenfragment darstellen,
oder R⁵ und R⁶ zusammen mit dem N-Atom einen Piperidino-, Morpholino- oder Piperazinorest bedeuten,
und die gewellte Bindung in der Amidinohydrazonstruktur bedeutet, daß die Verbindungen als (Z)- oder (E)-Isomere oder als Isomerengemische vorliegen, sowie deren Salze mit einer oder mehreren physiologisch verträglichen Säuren wie Mineralsäuren, linearen oder verzweigten Alkan- oder Alkensäuren oder Alkansulfonsäuren mit bis zu 6 C-Atomen oder Arencarbonsäuren, wobei die organischen Säuren gegebenenfalls mit Halogen-, Amino-, Dialkylamino(mit bis zu 6-C-Atomen), Hydroxy- und Carboxy-Resten substituiert sind.

2. Verfahren zur Herstellung von Amidinohydrazonen nach Anspruch 1, dadurch gekennzeichnet, daß man von Benzo[*b*]furan abgeleitete Ketone der allgemeinen Formel II, 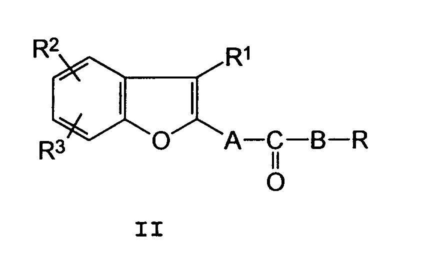
in der die Reste R, R¹, R² und R³ die Bedeutung gemäß Anspruch 1 haben,
mit einem Aminoguanidin der allgemeinen Formel III, 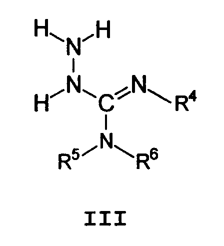
in der die Reste R⁴, R⁵ und R⁶ die Bedeutung gemäß Anspruch 1 haben, und wobei das Aminiguanidin gegebenenfalls als Salz mit einer physiologisch verträglichen anorganischen oder organischen Säure vorliegt,
durch Erhitzen in einem kurzkettigen Alkanol in Gegenwart von Mineral- bzw. Sulfonsäuren in an sich bekannter Weise kondensiert
und gegebenenfalls aus den so erhaltenen Verbindungen die Base freisetzt und diese Base entweder mit einer oder mehreren physiologisch vertretbaren Säuren wie Mineralsäuren, linearen oder verzweigten Alkan- oder Alkensäuren oder Alkansulfonsäuren mit bis zu 6 C-Atomen oder Arencarbonsäuren, wobei die organischen Säuren gegebenenfalls mit Halogen-, Amino-, Dialkylamino(mit bis zu 6-C-Atomen), Hydroxy- und Carboxy-Resten substituiert sind, umsetzt und in das physiologisch annehmbare Salz überführt,
oder in einem geeigneten Lösemittel, beispielsweise Pyridin, in Gegenwart von Hilfsbasen mit Acylhalogeniden der Formeln 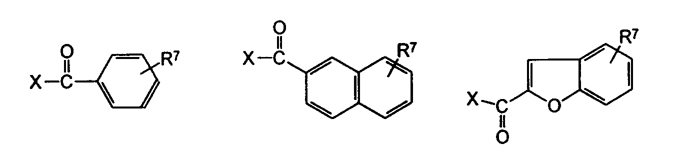 in denen R⁷ die in Anspruch 1 genannte Bedeutung hat und X ein Halogenatom bedeutet, umsetzt und die erhaltenen N-Acylderivate mit einer oder mehreren physiologisch vertretbaren Säuren wie Mineralsäuren, linearen oder verzweigten Alkan- oder Alkensäuren oder Alkansulfonsäuren mit bis zu 6 C-Atomen oder Arencarbonsäuren, wobei die organischen Säuren gegebenenfalls mit Halogen-, Amino-, Dialkylamino(mit bis zu 6-C-Atomen), Hydroxy- und Carboxy-Resten substituiert sind, umsetzt und in das physiologisch annehmbare Salz überführt.

3. Arzneimittel, gekennzeichnet durch den Gehalt an mindestens einer Verbindung gemäß dem Anspruch 1 sowie gegebenenfalls weiteren Wirkstoffen und zusammen mit pharmazeutisch verträglichen Hilfs- und Trägerstoffen.
